(19) **Europäisches Patentamt / European Patent Office / Office européen des brevets**

(11) **EP 3 833 777 B1**

(12) **EUROPEAN PATENT SPECIFICATION**

(45) Date of publication and mention of the grant of the patent:
**07.01.2026 Bulletin 2026/02**

(21) Application number: **19847000.7**

(22) Date of filing: **06.08.2019**

(51) International Patent Classification (IPC):
*C12Q 1/68* (2018.01)   *G06T 7/00* (2017.01)
*G16B 40/00* (2019.01)   *G06V 10/80* (2022.01)
*G06V 10/82* (2022.01)

(52) Cooperative Patent Classification (CPC):
**G06V 10/806; G06N 3/045; G06N 3/0455;
G06N 3/0464; G06N 3/048; G06N 3/09;
G06N 3/096; G06T 7/0012; G06V 10/82;
G16B 25/10; G16B 40/00; G16H 30/20;
G16H 30/40; G16H 40/67; G16H 50/20;** (Cont.)

(86) International application number:
**PCT/US2019/045368**

(87) International publication number:
**WO 2020/033453 (13.02.2020 Gazette 2020/07)**

(54) **A MULTI-MODAL APPROACH TO PREDICTING IMMUNE INFILTRATION BASED ON INTEGRATED RNA EXPRESSION AND IMAGING FEATURES**

MULTIMODALER ANSATZ ZUR PRÄDIKTION DER IMMUNINFILTRATION AUF DER GRUNDLAGE VON INTEGRIERTER RNA-EXPRESSION UND BILDGEBUNGSMERKMALEN

MÉTHODE MULTIMODALE POUR PRÉDIRE UNE INFILTRATION IMMUNITAIRE SUR LA BASE D'UNE EXPRESSION D'ARN INTÉGRÉE ET DE CARACTÉRISTIQUES D'IMAGERIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.08.2018 US 201862715079 P**

(43) Date of publication of application:
**16.06.2021 Bulletin 2021/24**

(73) Proprietor: **Tempus AI, Inc.**
**Chicago, IL 60654 (US)**

(72) Inventors:
• **LAU, Denise**
  **Chicago, IL 60615 (US)**
• **KHAN, Aly, Azeem**
  **Chicago, IL 60605 (US)**

(74) Representative: **Lucke, Andreas**
**Boehmert & Boehmert**
**Anwaltspartnerschaft mbB**
**Pettenkoferstrasse 22**
**80336 München (DE)**

(56) References cited:
**WO-A1-2018/091486   WO-A1-2019/020556
WO-A1-2019/108888   US-A1- 2014 017 227
US-A1- 2015 342 560   US-A1- 2017 175 199
US-A1- 2018 129 911**

• **POOYA MOBADERSANY ET AL: "Predicting cancer outcomes from histology and genomics using convolutional networks", PROCEEDINGS OF THE NATIONAL ACADEMY OF SCIENCES, vol. 115, no. 13, 12 March 2018 (2018-03-12), pages E2970 - E2979, XP055722606, ISSN: 0027-8424, DOI: 10.1073/pnas.1717139115**

**(Cont. next page)**

• SAFOORA YOUSEFI ET AL: "Predicting clinical outcomes from large scale cancer genomic profiles with deep survival models", SCIENTIFIC REPORTS, vol. 7, no. 1, 15 September 2017 (2017-09-15), XP055619027, DOI: 10.1038/ s41598-017-11817-6
• REIMAN DEREK ET AL: "Integrating RNA expression and visual features for immune infiltrate prediction", PACIFIC SYMPOSIUM ON BIOCOMPUTING. PACIFIC SYMPOSIUM ON BIOCOMPUTING, 23 January 2019 (2019-01-23), United States, pages 284 - 295, XP055904398, Retrieved from the Internet <URL:https://web. archive.org/web/20190204074217id_/http://psb. stanford.edu:80/psb-online/proceedings/psb19/ reiman.pdf> [retrieved on 20220323]
• SALTZ ET AL.: "Spatial Organization and Molecular Correlation of Tumor-Infiltrating Lymphocytes Using Deep Learning on Pathology Images", CELL REPORTS, vol. 23, no. 1, 3 April 2018 (2018-04-03), pages 181 - 193, XP055574630

• KARPINSKI ET AL.: "Immunological landscape of consensus clusters in colorectal cancer", ONCOTARGET, vol. 8, no. 62, 27 October 2017 (2017-10-27), pages 105299 - 105311, XP055683316

(52) Cooperative Patent Classification (CPC): (Cont.)
**G16H 50/30; G16H 50/70;** G06N 3/044;
G06N 20/10; G06T 2207/10024; G06T 2207/10056;
G06T 2207/20076; G06T 2207/20081;
G06T 2207/20084; G06T 2207/30024;
G06T 2207/30096; G06V 2201/03

**Description**

**Cross-Reference to Related Applications**

This application claims priority to U.S. Provisional Application No. 62/715,079, filed August 6, 2018.

**Field of the Invention**

The present disclosure relates to inferring the immune cell composition of a tumor sample and, more particularly, to predicting immune infiltration based on integrating multiple laboratory-based feature data.

**Background**

The background description provided herein is for the purpose of generally presenting the context of the disclosure. Work of the presently named inventors, to the extent it is described in this background section, as well as aspects of the description that may not otherwise qualify as prior art at the time of filing, are neither expressly nor impliedly admitted as prior art against the present disclosure.

Immune infiltration and its spatial organization within the tumor microenvironment have long been associated with cancer progression and clinical outcome. The potential of the immune infiltration as a prognostic biomarker has become increasingly relevant with the advent of cancer immunotherapies. Checkpoint blockade and other cancer immunotherapies, for example, can induce clinical responses in some cancer patients. Checkpoint blockade therapy and other cancer immunotherapies have shown striking clinical success in a wide range of malignancies, particularly those with melanoma, lung, bladder, and colorectal cancers. However, clinical responses are only observed in a portion of patients and vary for different cancer types, suggesting that additional factors such as the composition of the immune infiltrate may be important determinants of clinical response.

Several clinical studies have shown that the tumor immune microenvironment, particularly the presence or absence of key effector cells, such as cytotoxic CD8 T cells, can affect tumor immune responses. For example, solid tumors are commonly infiltrated by adaptive and innate immune cells, including T and B lymphocytes, natural killer (NK) cells, and macrophages (MACs). In the prevailing conventional model, distinct effector cells in the tumor-immune microenvironment cooperate to present, recognize, and respond to tumor-specific antigens. However, several roadblocks exist for routine, accurate and widespread pathological reporting of the immune infiltrate in tumor biopsies. Visual assessment after immunohistochemistry (IHC) staining for lineage specific markers remains the gold standard for evaluating immune cell infiltration in solid tumors; however, routine assessment is not possible due to the need for additional tissue samples and pathologist scoring of tissue slides. Alternatively, advances in genomic sequencing have facilitated implementation of RNA-sequencing (RNA-seq) in clinical medicine, but due to the inherent difficulty in deconvolving gene expression measurements into component immune cells, these approaches encounter significant ambiguity in reliably identifying correct immune proportions. Finally, emergent laboratory-based techniques, such as multiplex immunofluorescence, indexed flow cytometry, and single cell RNA-seq, require specialized labs and expertise, which has limited widespread access.

There is a need for techniques to accurately characterize the immune infiltrate in cancer patients in reproducible and cost effective ways, and to provide new prognostic markers.

Mobadersany, Pooya, et al. "Predicting cancer outcomes from histology and genomics using convolutional networks." Proceedings of the National Academy of Sciences 115.13 (2018): E2970-E2979 discloses a computational approach for learning patient outcomes from digital pathology images using deep learning to combine the power of adaptive machine learning algorithms with traditional survival models.

SAFOORA YOUSEFI ET AL, "Predicting clinical outcomes from large scale cancer genomic profiles with deep survival models", SCIENTIFIC REPORTS, (20170915), vol. 7, no. 1, doi:10.1038/s41598-017-11817-6 * Section "Comparing deep survival networks with Cox elastic net and random survival forests" discloses a comparison of the performance of SurvivalNet models with Cox elastic net (CEN) and random survival forest (RSF) models using data from multiple TCGA projects.

**Summary of the Invention**

The present application presents a multi-modal approach to predicting immune infiltration based on integrating gene expression data and imaging features. In some examples, that gene expression data is RNA expression data. Indeed, the present approaches are able to use multiple laboratory-based modalities to predict immune infiltration in tumor samples. In exemplary embodiments two modalities are described: gene expression (such as RNA expression data) and imaging features. More broadly, however, any number of modalities can be combined with the described integration model. Examples of such additional modalities include contextual information, such as tumor information and data such as methylation sequencing data.

In exemplary embodiments, a machine learning framework integrates data from the different laboratory-based modalities and predicts immune cell infiltration based on the assessment of the integrated output from the framework. In some examples, the machine learning framework predicts immune cell infiltration based on an assessment of RNA expression data, or based on an assessment of RNA expression data integrated with imaging data, or based on an assessment of RNA expression data integrated with imaging data and other biological data derived from another laboratory-based modality, such as percentage of immune cells or percentage of tumor cells in a sample, or methylation sequencing data.

In accordance with an example, a computing device configured to generate an immune infiltration prediction score, the computing device comprising one or more processors is configured to: obtain gene expression data from one or more gene expression datasets with the gene expression data corresponding to one or more tissue samples; obtain a set of stained histopathology images from one or more image sources and corresponding to the one or more tissue samples; determine imaging features from the set of stained histopathology images, the imaging features comprising texture and/or intensity features; in a neural network-based framework, transform the gene expression data using a gene expression neural network layer(s) and transform the imaging features using an imaging feature neural network layer(s); in the neural network framework, integrate an output of the gene expression neural network layer(s) and the imaging feature neural network layer(s) to produce an integrated neural network output; and apply a prediction function to the integrated neural network output and output an immune infiltration score for the one or more tissue samples.

In accordance with an example, a computing device configured to generate an immune infiltration prediction score, the computing device comprising one or more processors configured to: obtain a gene expression data from one or more gene expression datasets with the gene expression data corresponding to one or more tissue samples; obtain a set of stained histopathology images from one or more image sources and corresponding to the one or more tissue samples; determine imaging features from the set of stained histopathology images, the imaging features comprising texture and/or intensity features; obtain contextual data corresponding to the one or more tissue samples; in a neural network-based framework, transform the gene expression data using a gene expression neural network layer(s), transform the imaging features using an imaging feature neural network layer(s), and transform the contextual data using contextual feature neural network layer(s); in the neural network framework, integrate an output of the gene expression neural network layer(s), the imaging feature neural network layer(s), and the contextual feature neural network layer(s) to produce an integrated neural network output; and apply a prediction function to the integrated neural network output and output an immune infiltration score for the one or more tissue samples.

In accordance with another example, a computing device configured to generate an immune infiltration prediction score, the computing device comprising one or more processors configured to: obtain a gene expression data from one or more gene expression datasets with the gene expression data corresponding to one or more tissue samples; in a neural network framework, transform the gene expression data using one or more neural network layers and apply a prediction function to the transformed gene expression data, the prediction function implemented using one or more neural network layers, the output of the prediction function being an immune infiltration score for the one or more tissue samples.

In accordance with some examples, the gene expression data is RNA sequencing data.

In accordance with some examples, the neural network framework comprises two neural network layers.

In accordance with some examples, the imaging features comprise mean, standard deviation, skewness, and/or sum of image gray level, image red, green, blue layers, stain layers, optical density, hue, and/or saturation.

In accordance with some examples, the imaging features comprise Zernike moments, threshold adjacency analysis values, local binary patterns, gray scale co-occurrence matrix, and/or difference of Gaussian statistical measures.

In accordance with some examples, the prediction function is Softmax function.

In accordance with some examples, immune infiltration score comprises a predicted percentage of natural killer (NK) cells, (MAC) macrophage cells, CD4 T cells, CD8 T cells, and B cells, regulatory T cells, Dendritic cells, monocytes, Mast cells, Eosinophils, and Neutrophils.

In accordance with some examples, the immune infiltration score comprises a predicted percentage of others cells, including stromal cells, vasculature cells, fat cells, tumor cells, stem cells, neural cells, progenitor cells, innate lymphoid cells, microglial cells, leukocytes, naïve B cells, memory B cells, Plasma cells, CD8 T cells, naïve CD4 T cells, memory CD4 T cells, follicular helper T cells, regulatory T cells, gamma delta T cells, Th17 T cells, unstimulated NK cells, stimulated NK cells, Macrophages MO, Macrophages M1, Macrophages M2, unstimulated Dendritic cells, stimulated Dendritic cells, unstimulated Mast cells, stimulated Mast cells.

In accordance with some examples, the contextual data is a total immune fraction or total tumor fraction.

In accordance with an example, a computer-implemented method to generate an immune infiltration prediction score, the method comprises: obtaining a gene expression data from one or more gene expression datasets with the gene expression data corresponding to one or more tissue samples; obtaining a set of stained histopathology images from one or more image sources and corresponding to the one or more tissue samples; determining imaging features from the set of stained histopathology images, the imaging features comprising texture and/or intensity features; in a neural network framework, transforming the gene expression data using a gene expression neural network layer(s) and transforming the imaging features using an imaging feature neural network layer(s); in the neural network framework, integrating an output of the gene expression neural network layer(s) and the imaging feature neural network layer(s) to produce an integrated neural network output; and applying a prediction function to the integrated neural network output and outputting an immune infiltration score for the one or more tissue samples.

In accordance with an example, a computer-implemented method to generate an immune infiltration prediction score, the method comprises: obtaining a gene expression data from one or more gene expression datasets with the gene expression data corresponding to one or more tissue samples; obtaining a set of stained histopathology images from one or more image sources and corresponding to the one or more tissue samples; determining imaging features from the set of stained histopathology images, the imaging features comprising texture and/or intensity features; obtaining contextual data corresponding to the one or more tissue samples; in a neural network framework, transforming the gene expression data using a gene expression neural network layer(s), transforming the imaging features using an imaging feature neural network layer(s), and transforming the contextual data using contextual feature neural network layer(s); in the neural network framework, integrating an output of the gene expression neural network layer(s), the imaging feature neural network layer(s), and the contextual feature neural network layer(s) to produce an integrated neural network output; and applying a prediction function to the integrated neural network output and outputting an immune infiltration score for the one or more tissue samples.

In accordance with an example, a computer-implemented method to generate an immune infiltration prediction score, the method comprises: obtaining a gene expression data from one or more gene expression datasets with the gene expression data corresponding to one or more tissue samples; and in a neural network framework, transforming the gene expression data using one or more neural network layers and applying a prediction function to the transformed gene expression data; the prediction function implemented using one or more neural network layers, the output of the prediction function being an immune infiltration score for the one or more tissue samples.

In accordance with another example, a computing device is configured to predict the composition for a sample of a biological mixture of cells, the computing device comprising one or more processors configured to: obtain a gene expression data from one or more gene expression datasets; the gene expression data corresponding to the sample; obtain stained histopathology images from one or more image sources and corresponding to the sample; in a neural network framework having two or more neural network layers, integrate the gene expression data and the imaging features determined from the stained histopathology images; and apply a prediction function to the integrated output to predict cell composition for the sample, such as the estimated relative proportion of tumor and/or endothelial cells. In some examples, the predicted cell composition is used to assess a biological condition of the tissue, such as how much vascularization is present in a tumor.

In accordance with other examples, these techniques are not limited to cancer samples, but rather can be used to infer the relative and absolute proportions of different immune cell types and has value in many other disease areas, like lupus and rheumatoid arthritis.

## Brief Description of the Drawings

The figures described below depict various aspects of the system and methods disclosed herein. It should be understood that each figure depicts an example of aspects of the present systems and methods.

FIG. 1 is a schematic illustration of an example immune infiltration predictor processing system having a machine learning framework providing a multi-modal approach, e.g., based on integration of gene expression and imaging features, in accordance with an example.

FIG. 2 is a schematic Illustration of a machine learning framework that can be implemented by the immune infiltration predictor processing system of FIG. 1, in accordance with an example.

FIG. 3A is pipeline flow diagram of an implementation of the immune infiltration predictor processing system of FIG. 1 used to predict tumor immune infiltrate fraction and composition, in accordance with example. Alternating slides cut from primary tumor FFPE blocks were used for RNA-sequencing and H&E staining. RNA expression data and imaging features were inputted into immune infiltration predictor processing system. The immune infiltrate predictions from FIG. 3A were compared to pathologist expert review of stained tumor sections using a panel of immune lineage markers shown in FIG. 3B.

FIG. 4 illustrates two example pipelines for predicting relative immune proportion, one pipeline using RNA sequencing data and the other pipeline integration RNA sequencing data with imaging features, as may be implemented by the immune infiltration predictor processing of FIG. 1, in accordance with an example.

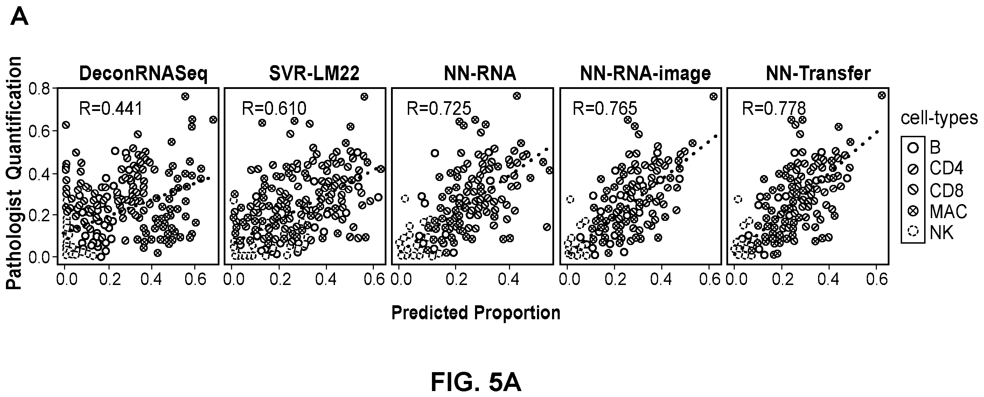

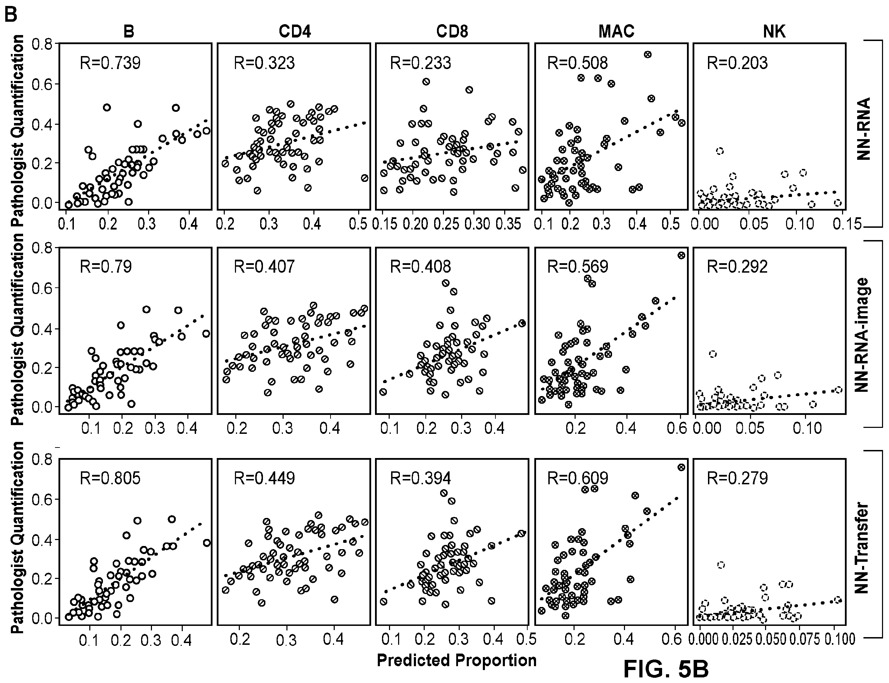

FIGS. 5A and 5B are plots showing model performance comparisons benchmarked compared to expert pathologist assessment. FIG. 5A provides plots of predicted proportions of B, CD4 T, CD8 T, MAC, and NK cells of five different models, with DeconRNASeq and SVR-LM22 being convention models and NN-RNA, NN-RNA-image, and NN-Transfer being example implementations of the present techniques, in comparison to pathologist scoring of IHC for lineage specific markers for 61 solid tumor samples. The sum of the proportions for all the cells for a particular sample equal 1. The color of each point denotes the cell-type and the dotted line represents the linear regression line that best fits the data. The value of the Pearson correlation coefficient is shown in upper left corner of each plot. FIG. 5B illustrates the same data as FIG. 5A for the three neural network-based models, but separated by immune cell-types.

FIG. 6 illustrates the two example pipelines of FIG. 4, along with a third example pipeline for predicting relative immune proportion using RNA sequencing data integrated with imaging features and total immune fraction data, as may be implemented by the immune infiltration predictor processing of FIG. 1, in accordance with an example.

FIG. 7 provides plots of benchmarking of a total immune infiltrate fraction determination using the pipeline of FIG. 6. The scatter plots illustrate pathologist counts compared to the immune score from ESTIMATE (left) and the predicted total fraction of immune infiltrate from NN-RNA-image (right).

FIG. 8 is a block diagram of example method for performing an NN-RNA pipeline to predict immune infiltration as implemented by the immune infiltration predictor processing system of FIG. 1, in accordance with an example.

FIG. 9 is a block diagram of example method for performing a first multi-modal technique to predict immune infiltration (specifically an example NN-RNA-image pipeline) as implemented by the immune infiltration predictor processing system of FIG. 1, in accordance with an example.

FIG. 10 is a block diagram of example method for performing a second multi-modal technique to predict immune infiltration (specifically an example NN-Transfer pipeline) as implemented by the immune infiltration predictor processing system of FIG. 1, in accordance with an example.

FIG. 11 illustrates an example computing device for implementing the systems of FIGS. 1 and 2 and the processes of FIGS. 3 and 8-10, in accordance with an example implementation.

## Detailed Description

The present application presents a multi-modal approach to predicting immune infiltration based on integrating gene expression data and imaging features. In some examples, that gene expression data is RNA sequencing expression data. Indeed, the present approaches are able to use multiple laboratory-based modalities to predict immune infiltration in tumor samples. In exemplary embodiments two modalities are described: RNA expression and imaging features. More broadly, however, any number of modalities can be combined with the described integration model. Examples of such additional modalities include contextual tumor information and data such as methylation sequencing data.

In exemplary embodiments, a machine learning framework is used to integrate the data from different laboratory-based modalities and predict immune cell infiltration based on the assessment of the integrated output from the framework. In some examples, the machine learning framework predicts immune cell infiltration based on an assessment of RNA expression data, or based on an assessment of RNA expression data integrated with imaging data, or based on an assessment of RNA expression data integrated with imaging data and another laboratory-based modality, for example, contextual information, such as percentage of immune cells or percentage of tumor cells in a sample, or methylation sequencing data.

In exemplary arrangements, neural-network based frameworks are designed for integrating gene expression and visual imaging features (including intensity and texture features) to accurately model immune infiltration in solid tumors. We demonstrate the utility of such frameworks as capable of predicting immune infiltrates across different cancer types and evaluated our system predictions against expert pathology review. Our analysis demonstrates that integration of imaging features greatly improves prediction of the immune infiltrate in comparison to conventional techniques. The frameworks exhibit improved efficacy across immune cells, including natural killer (NK), (MAC) macrophage, CD4 T cells, CD8 T cells, and B cells. In some examples, the scoring output by the framework may include percentage of others cells, including stromal cells, vasculature cells, fat cells, tumor cells, stem cells, neural cells, progenitor cells, innate lymphoid cells, microglial cells, leukocytes, naïve B cells, memory B cells, Plasma cells, CD8T cells, naïve CD4T cells, memory CD4Tcells, follicular helper Tcells, regulatory Tcells, gamma delta Tcells, Th17 Tcells, unstimulated NK cells, stimulated NK cells, Monocytes, Macrophages MO, Macrophages M1, Macrophages M2, unstimulated Dendritic cells, stimulated Dendritic cells, unstimulated Mast cells, stimulated Mast cells, Eosinophils, and Neutrophils.

In exemplary embodiments, the present techniques include integrating (i) coarse visual texture features from medical images, such as routine hematoxylin and eosin (H&E) stained images of solid tumors used in cancer staging and diagnosis, with (ii) bulk tumor RNA-Seq data to reduce ambiguity in predicting the immune infiltrate. In exemplary embodiments, the techniques are implemented through a framework configured with a neural network-based approach for integrating this gene expression data with the visual texture features from solid tumor samples in a clinical laboratory setting.

In exemplary embodiments, the frameworks effectively integrate both gene expression data (e.g., RNA sequencing (RNA-Seq) data) and imaging data in a clinical setting and provide a more reliable and accurate prediction of the immune composition in individual patient tumors. In the example of RNA expression data, the RNA expression data may be RNA-seq data obtained from an RNA sequencer, such as from a so called Next Gen Sequencer (NGS) machine. The RNA-seq data may be obtained from a gene expression database, such as the Encyclopedia of DNA Elements (ENCODE) project databases, The Cancer Genome Atlas (TCGA) project databases, and the Genotype-Tissue Expression (GTEx) program databases.

In exemplary embodiments, the techniques are used to predict both relative proportion of individual key effector immune cells and total fraction of the tumor immune infiltrate. In particular, owing to flexibility configured into the present neural network-based approaches, we are able to evaluate the integration of additional contextual features, such as estimates of the total fraction of immune infiltrate, to score immune infiltration, in some examples.

In some examples, the present techniques provide a system for analyzing medical images for a patient. Those medical images may be any stained medical image, such as histopathology slide images or other medical images of stained cells. These histopathology images may be collected from tissue captured *in vivo* or *ex vivo,* and may be stained with H&E or immunofluorescence or immunohistochemistry stains.

The tumor microenvironment is extremely complex and heterogeneous, containing a diverse mixture of tumor, stromal, and immune cells. Tumors use immune checkpoints in the body to shield themselves from attack by the immune system. These immune checkpoints can be stimulatory or inhibitory, and checkpoint blockade therapies are being developed to stop this tumor shielding process. The success of checkpoint blockade treatment is based, in part, on the activation of infiltrating immune cells present in the tumor microenvironment. Information about infiltrating immune cells in histopathology slides is normally only accessible by overlaying additional multiplexed immunofluorescence or immunohistochemistry stains.

In exemplary embodiments, an imaging feature module examines microscopic stained images (e.g., histopathology slides) for fine-grained information, or features, of the topology, morphology, and population structure of cells within the tumor-immune microenvironment. In exemplary embodiments, these stained medical images are examined in patches or tiles that represent groups of pixels, and image features are determined from these patches. These image features include, by way of example, image intensity features such as the mean, standard deviation, skewness, and sum, where applicable, for image gray level; image red, green, blue layers; H&E stain layers; optical density (od) 3 channels; hue; and saturation. The image features include texture features including Zernike moments (0-24 moments), threshold adjacency analysis values (statistics 0-53), local binary patterns (histogram bins 0-25), gray scale co-occurrence matrix, and difference of Gaussian statistical measures. Image features may also be generated in a non-directed manner through the use of an autoencoder, or the decoder portion or the encoder portion of an autoencoder.

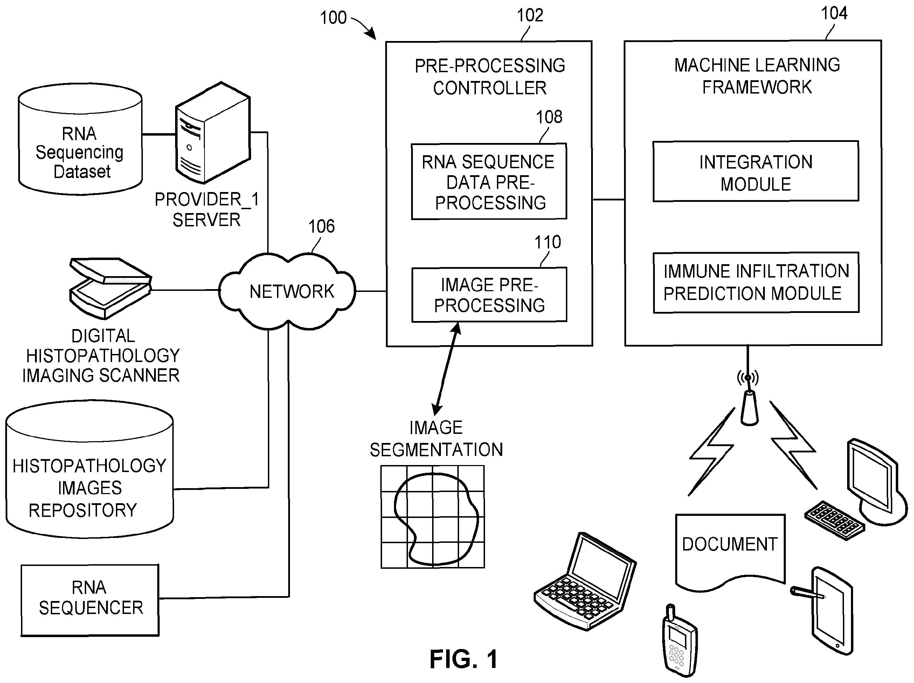

FIG. 1 illustrates an immune infiltration predictor processing system 100 for predicting immune infiltration in cells for a patient via analysis of RNA sequencing features, imaging features, and other laboratory-based modality data.

The immune infiltration processing system 100 includes a pre-processing controller 102 and a machine framework 104. The pre-processing controller 102 is communicatively coupled to a network 106 and receives medical images (e.g., any stained medical images such as H&E stained histopathology images) from a variety of different sources, including (i) dedicated digital medical image scanners which may be any suitable optical histopathology slide scanner including 20x and 40x resolution magnification scanners and (ii) histopathology image repositories, such as the Cancer Genome Atlas (TCGA) and NCI Genomic Data Commons. The pre-processing controller 102 also receives RNA sequencing data, for example, from (i) a dedicated RNA sequencer station and (ii) an RNA sequencing dataset from a healthcare provider (Provider_1) such as a hospital, physician group, lab, etc. Each of the image sources and RNA sequencing sources may present multiple sources. Example RNA sequencing datasets that may be communicatively coupled to the network 106 include the Cancer Genome Atlas (TCGA) and the Genotype-Tissue Expression (GTEx) Program database. Further, while RNA sequencing data sources are shown, these sources represent more generally any gene expression dataset source.

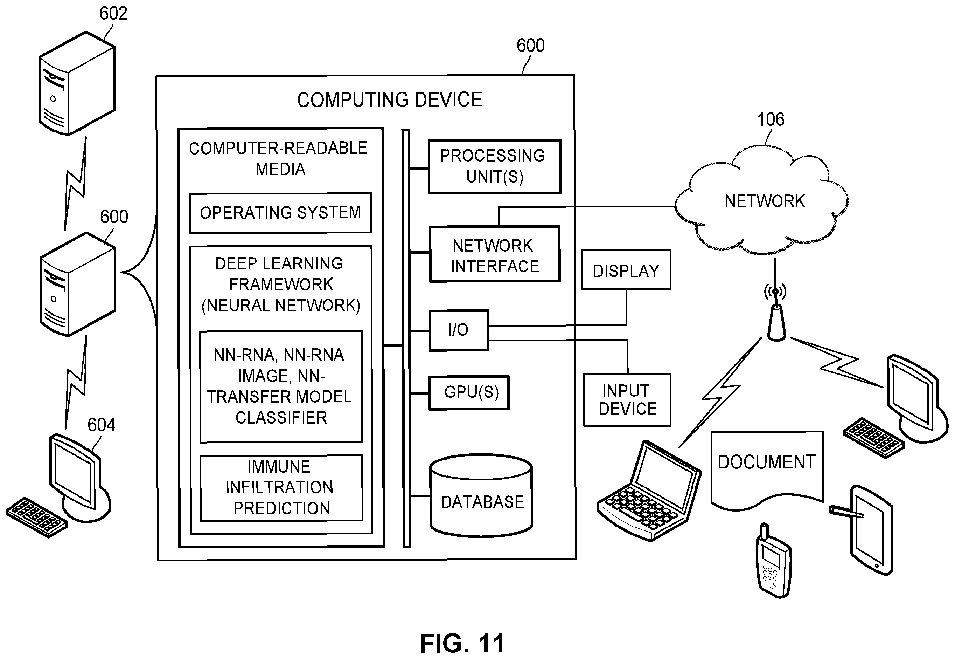

The immune infiltration predictor processing system 100 may be implemented on a computing device such as a computer, tablet or other mobile computing device, or server. The system 100 may include a number of processors, controllers or other electronic components for processing or facilitating the RNA sequencing feature analysis, imaging feature analysis, other modality feature analysis, and immune infiltration analysis, as described herein. An example computing device 600 for implementing the immune infiltration predictor processing system 100 is illustrated in FIG. 11. As illustrated, the system 100 may be implemented on the computing device 600 and in particular on one or more processing units, which may represent Central Processing Units (CPUs), and/or on one or more or Graphical Processing Units (GPUs), including clusters of CPUs and/or GPUs. Features and functions described for the system 100 may be stored on and implemented from one or more non-transitory computer-readable media of the computing device 600. The computer-readable media may include, for example, an operating system, a pre-processing controller, and a machine framework having elements corresponding to that of immune infiltration predictor processing system 100, including an RNA sequencing feature module, an imaging feature module, a neural network based integration module, and an immune infiltration module. More generally, the computer-readable media may store trained machine learning models, executable code, etc. use for implementing the techniques herein. The computing device 600 includes a network interface communicatively coupled to the network 106, for communicating to and/or from a portable personal computer, smart phone, electronic document, tablet, and/or desktop personal computer, or other computing devices. The computing device further includes an I/O interface connected to devices, such as digital displays, user input devices, etc.

In the illustrated example, the immune infiltration predictor processing system 100 is implemented on a single server 600. However, the functions of the system 100 may be implemented across distributed devices 600, 602, 604, etc. connected to one another through a communication link. In other examples, functionality of the system 100 may be distributed across any number of devices, including the portable personal computer, smart phone, electronic document, tablet, and desktop personal computer devices shown. The network 106 may be a public network such as the Internet, a private network such as that of research institution or a corporation, or any combination thereof. Networks can include, local area network (LAN), wide area network (WAN), cellular, satellite, or other network infrastructure, whether wireless or wired. The network can utilize communications protocols, including packet-based and/or datagram-based protocols such as Internet protocol (IP), transmission control protocol (TCP), user datagram protocol (UDP), or other types of protocols. Moreover, the network can include a number of devices that facilitate network communications and/or form a hardware basis for the networks, such as switches, routers, gateways, access points (such as a wireless access point as shown), firewalls, base stations, repeaters, backbone devices, etc.

The computer-readable media may include executable computer-readable code stored thereon for programming a computer (e.g., comprising a processor(s) and GPU(s)) to the techniques herein. Examples of such computer-readable storage media include a hard disk, a CD-ROM, digital versatile disks (DVDs), an optical storage device, a magnetic storage device, a ROM (Read Only Memory), a PROM (Programmable Read Only Memory), an EPROM (Erasable Programmable Read Only Memory), an EEPROM (Electrically Erasable Programmable Read Only Memory) and a Flash memory. More generally, the processing units of the computing device 600 may represent a CPU-type processing unit, a GPU-type processing unit, a field-programmable gate array (FPGA), another class of digital signal processor (DSP), or other hardware logic components that can be driven by a CPU.

Returning to FIG. 1, the image processing controller 102 includes a RNA sequencing pre-processing module 108 and an image pre-processing module 110.

The RNA sequencing pre-processing module 108 may perform RNA normalization and error correction. Because RNA expression may come from different batches or sequencers, in some example the controller module 108 performs batch correction. Additionally, in some example, the module 108 performs RNA expression normalization where values are changed to remove instrument, or sequencing adapter or other laboratory biases. In some examples, the module 108 deploys a neural network to perform undirected RNA normalization and transformation.

The image pre-processing module 110 may perform initial processes such as image quality assessment and noise filtering. Because medical images such as H&E stained histopathology images may come from different sources, including different scanners and scanner types, in some examples the controller module 110 performs a color normalization to establish a uniform color-scape for the medical images. Additionally, in some examples, the module 110 performs a tissue segmentation that identifies target tissue within the received medical images and segments that target tissue for analysis by the machine learning framework 104. In some examples, the module 110 deploys a convolutional neural network to perform whole slide image segmentation; although any number of unsupervised or supervised methods of image segmentation may be used.

In exemplary embodiments, the tissue segmentation process identifies patches that will be used for analysis by the machine learning framework 104. Patches may be geometric, e.g., a repeating pattern of square or rectangular pixels defined across each medical image and at a pixel size sufficient to analyse changes in topology and morphology in medical images. Example patch sizes include 1000x1000 pixels, although fewer pixels can be used, such as 900x900, 800x800, 700x700, 600x600, 500x500, and so on, down to at least 100x100, and even further, such as 50X50 depending on the application. In other examples, patch type may be non-geometric, also termed herein a "super pixel," where each patch is allowed to vary in shape, but where the super pixels are generated to include a sufficient threshold of imaging information for topology and morphology analysis.

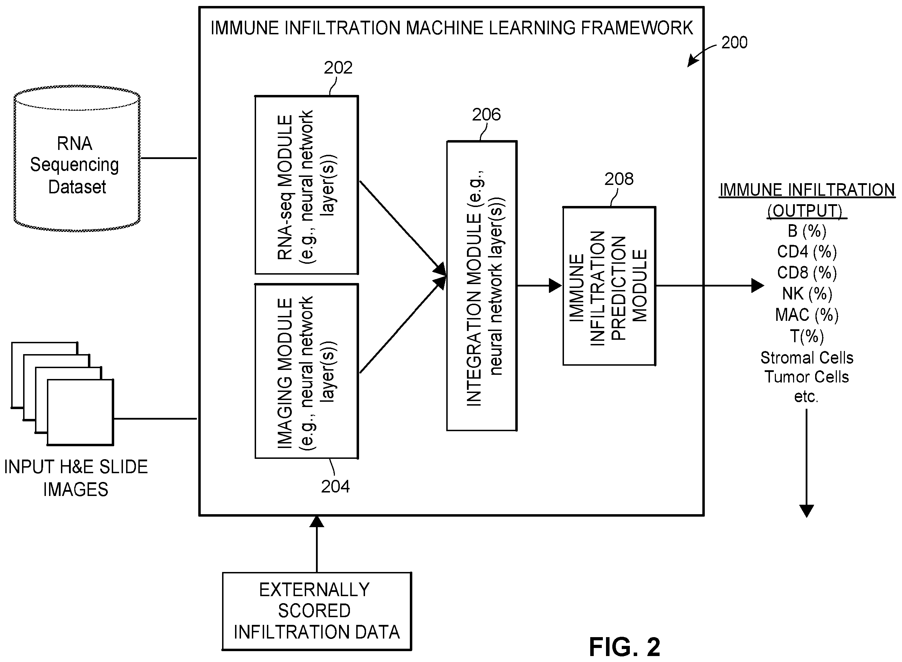

FIG. 2 illustrates an example framework 200 for implementing the machine learning framework 104. Gene expression data is received via a dataset, in the illustrated example, via an RNA sequencing dataset (e.g., from the pre-processing controller 108). Digital H&E slide images are provided (e.g., from the pre-processing controller 110) to the framework 200. Score infiltration data for the H&E slide images is provided to the framework 200, as well. That score infiltration data may be data scored by a pathologist or data that has been scored by automated computer processing to identify immune infiltration in the image data.

An RNA features module 202 receives the RNA sequencing data, whether pre-processed or not, as raw RNA sequencing data. The RNA module 202 processes the raw RNA expression data by performing directed gene selection or automatically transforming the RNA expression data using or more neural network layers. Example neural network implementations include autoencoder and feed forward networks. An autoencoder is a neural network used to learn efficient data codings in an unsupervised manner, to reduce dimensionality of the dataset. Feed forward neural networks are neural networks where connections between the nodes do not form a cycle, in comparison to recurrent neural networks, such that classification data moves in one direction. Such neural networks may be single layer or multi-layer.

The imaging features module 204 receives raw imaging data and processes that data by performing directed feature extraction or automatically transforming imaging data using one or more neural networks, such as an autoencoder or feed forward neural network, as in the RNA features module 202.

The outputs of the RNA features module 202 and the imaging features module 204 are provided to a machine learning integration module 206 that integrates these two different modes of biological data. In other examples, the machine learning integration module 206 integrates any number of different biological data, from any number of laboratory-based modalities. The machine learning integration module 206 may be implemented by a Neural Network, Support Vector Machine (SVM), boosting, or other machine learning process. In some examples, the machine learning integration module is neural network with one or more neural network layers.

The output of the integration module 206 is provided to an immune infiltration prediction module 208 that stores a trained model that, upon receipt and analysis of new biological data, outputs an immune infiltration score for that biological data. The immune infiltration score, for example, may be a percentage of predicted immune cells based on the received biological data. In exemplary embodiments, the score is a percentage of immune cells, T cells, B cells, CD4 cells, CD8 cells, neutral killer (NK) cells, and (MAC) macrophage cells. The score may include percentage of others cells, including stromal cells, vasculature cells, fat cells, tumor cells, stem cells, neural cells, progenitor cells, innate lymphoid cells, microglial cells, leukocytes, naïve B cells, memory B cells, Plasma cells, CD8 T cells, naïve CD4 T cells, memory CD4 T cells, follicular helper T cells, regulatory T cells, gamma delta T cells, Th17 T cells, unstimulated NK cells, stimulated NK cells, Monocytes, Macrophages MO, Macrophages M1, Macrophages M2, unstimulated Dendritic cells, stimulated Dendritic cells, unstimulated Mast cells, stimulated Mast cells, Eosinophils, and Neutrophils.

## Example

In an example implementation of the immune infiltration predictor processing system 100 of FIG. 1 machine learning framework 200 of FIG. 2, we examined numerous solid tumor blocks in a pipeline that combined RNA sequencing features, visual texture features, and immunochemistry contextual data, to predict immune infiltration.

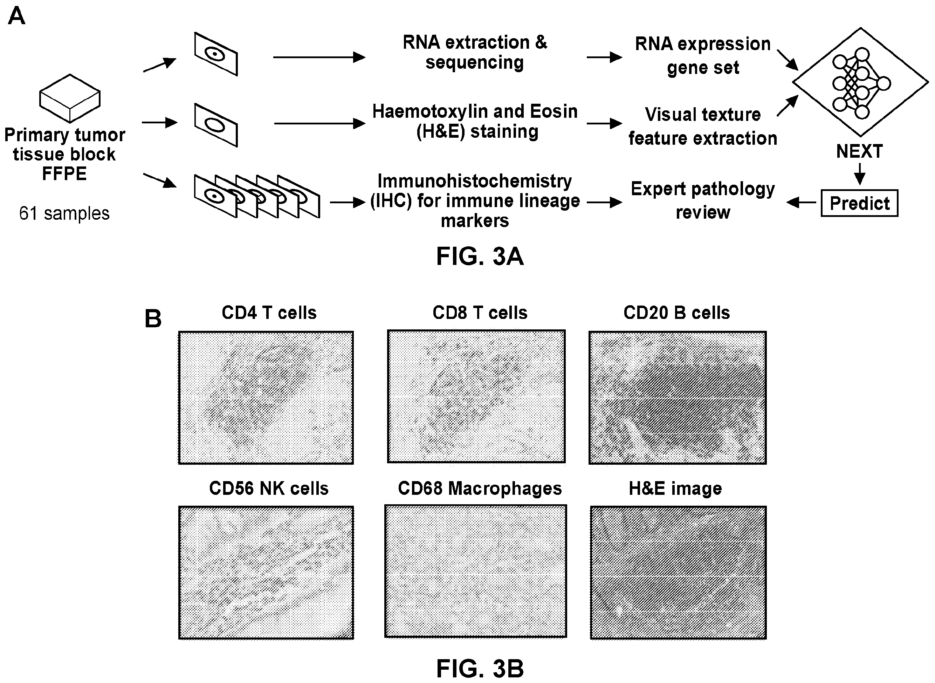

In an experiment, 61 formalin-fixed paraffin-embedded (FFPE) solid tumor blocks (specifically colorectal (n = 14), breast (n = 15), lung (n = 17) and pancreatic (n = 15)) were cut into alternating sections for RNA sequencing data, hematoxylin and eosin (H&E) staining data, and immunohistochemistry (IHC) staining data as shown in FIG. 3A. For the RNA sequencing data pipeline, the RNA module obtained normalized read counts from the RNA-seq data for a specific panel of genes. For the image data pipeline, the imaging features module generated visual texture features from H&E stained slides. Feature data from both pipelines were combined and analysed by the machine learning framework, which generated immune infiltration predictions that were compared to pathologist expert review of IHC stained tumor sections (in a third, manual pipeline), using a panel of immune lineage markers (FIG. 3B).

To perform the RNA extraction and sequencing, total nucleic acid was extracted from the FFPE tumor tissue sections, macrodissected based on pathologist assessment of tumor cellularity, and proteinase K digested. Total nucleic acid was extracted with a Chemagic360 instrument using a source-specific magnetic bead protocol and stored at 4°C if less than 24 hours and -80°C if longer. RNA was purified from the total nucleic acid by DNase I digestion and magnetic bead purification. RNA from both sources was quantified by a Quant-iT picogreen dsDNA reagent Kit or Quant-iT Ribogreen RNA Kit (available from Life Technologies, Inc. of Carlsbad, CA). Quality was confirmed using a LabChip GX Touch HT Genomic DNA Reagent Kit or LabChip RNA High HT Pico Sensitivity Reagent Kit (available from PerkinElmer of Waltham, MA).

RNA libraries were prepared using the KAPA RNA HyperPrep Kit. One hundred nanograms of RNA per tumor sample were fragmented with heat in the presence of magnesium to an average size of 200 base pairs. RNA underwent first strand cDNA synthesis using random primers, followed by combined second strand synthesis, A-tailing, adapter ligation, bead-based cleanup, and library amplification. After library preparation, samples were hybridized with the IDT xGEN Exome Research Panel. Target recovery was performed using Streptavidin-coated beads, followed by amplification using the KAPA HiFi Library Amplification Kit. The RNA libraries were sequenced to obtain an average of 90 million reads, minimum of 50 million reads, on an Illumina HiSeq 4000 System utilizing patterned flow cell technology.

The RNA sequencing data was stored in FASTQ files, a text-based format storing a RNA sequencing data and its corresponding quality scores, were uploaded to a centralized server (in this example an Amazon Web Services (AWS)) following completion of sequencing. The analytical processing was triggered via the JANE workflow orchestration tool (Tempus Labs, Inc. of Chicago, IL) and analyzed using the CRISP clinical RNA-seq pipeline. CRISP performs pre-alignment QC, read grooming, alignment, post-align QC, and gene level quantification. The gene level counts from CRISP are then converted to TPMs (transcripts per million) to normalize for gene length and library size.

The resulting RNA sequencing data had now been pre-processed and extracted and ready for application to a machine learning integration module.

For the image feature extraction, during image pre-processing, (H&E) stained slide images were tiled and downsampled, generating overlapping square tiles (also termed "patches" herein) with 210x210 microns in tile size and 30 microns in shifting strip size. Image tiles were down-sampled by 4 on each edge, as 1 micron equals 4 pixels in size.

Statistical features for each tile were generated and converted into 196 feature vectors, formed of intensity features and texture features.

The image intensity features include the mean, standard deviation, skewness, and sum, where applicable, for the gray level; red, green, blue layer; H&E stain layers; optical density (od) 3 channels; hue; and saturation. The visual texture features included Zernike moments (0-24 moments), threshold adjacency analysis values (statistics 0-53), local binary patterns (histogram bins 0-25), gray scale co-occurrence matrix and difference of Gaussian statistical measures.

To achieve immunohistochemistry staining for lineage specific markers, all FFPE slides were stained using the Leica Bond III automated IHC instrument and Leica reagents. The Leica antibody panel included: CD45 clone X16/99, CD4 clone 4B12, CD8 clone 4B11, CD20 clone L26, CD56 clone CD564, and CD68 clone 514H12. CD20 was used in a 1:200 dilution, but all other antibodies were purchased prediluted. Slides were deparaffinized using Dewax Solution. Heat induced epitope retrieval was used to reverse cross-linked proteins in the tissue by heating slides to 38 degrees Celsius and applying Epitope Retrieval Solution 1, a citrate-based solution with a pH of 6.0. The Bond Polymer Refine Detection kit was used for IHC staining and hematoxylin counterstaining. Slides were then dehydrated, mounted, and cover-slipped.

To provide a gold standard comparison, in this experiment, expert pathology review of histology slides was performed. The IHC and H&E stained slides were scored by a pathologist. The percent of each immune cell-type of interest (CD20+ B, CD4+ T, CD8+ T, CD68 MAC, CD56 NK cells) and total immune percentage (CD45) was determined by estimating the percent of cells that stained positive by IHC for the protein uniquely expressed by that cell-type. The pathologist was instructed to exclude staining of non-immune cells in their scoring. For instance, if 20% of all cells on a slide stained positively for CD20 B cells, but half of those positively staining cells were tumor cells, that sample would be scored as having 10% B cells. The percent tumor, stroma, and immune cells were estimated from evaluating the cell morphologies on their respective H&E slides. The relative abundance of the immune cell-types was determined by dividing the percent of the particular cell-type by the percent of total immune cells.

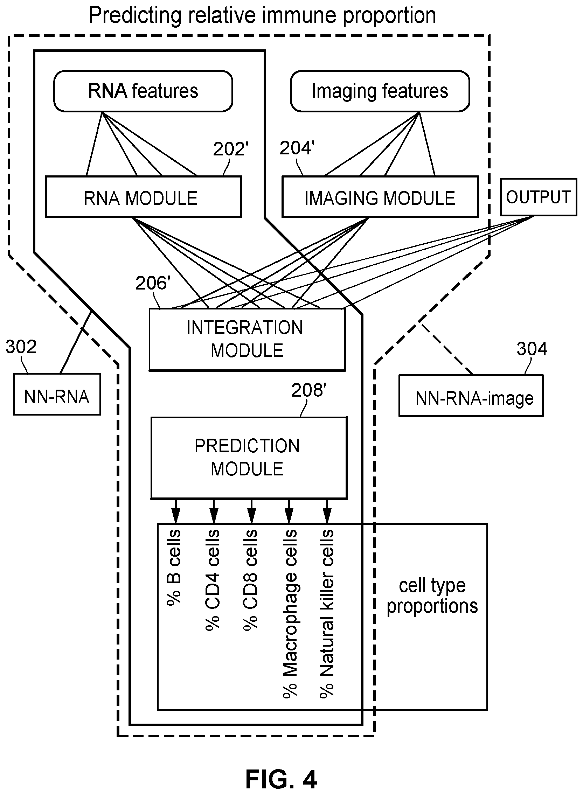

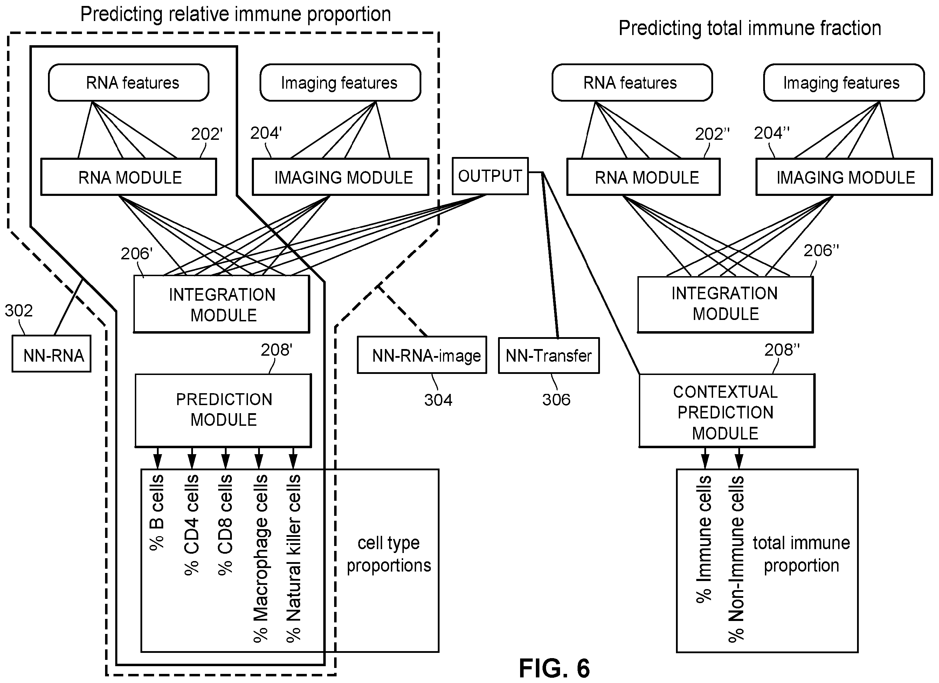

In exemplary embodiments, the machine learning implementations herein may be achieved using neural networks. Neural networks can function as flexible and efficient learning models when integrating heterogeneous features, such as gene expression and imaging features. Thus, in some examples, the machine learning framework uses a neural network-based architecture to integrate RNA-seq and imaging data. Indeed, we used the machine learning framework in three separate architectures: NN-RNA (FIG. 4), NN-RNA-image (FIG. 4), and NN-Transfer (FIG. 6).

In an embodiment of FIG. 4, the machine learning framework is formed of a shallow neural network that consists of 3 or less layers containing a set of neurons, where each neuron is a weighted sum of the inputs in that layer. In an implementation of the similar elements of FIG. 2, the RNA sequence module 202', the imaging module 204', and the integration module 206', are each formed of a single neural network layer, e.g., having 32 nodes, 64 nodes, or 128 nodes, depending on the number of samples. Any of these modules may include more than one neural network layer, in other configurations. In any event, for the example shown, non-linear activation functions are applied to the neurons to allow the model to find non-linear relationships between gene expression and imaging features, for example. The output of a layer is then used as the input to the next layer. More specifically, given an input vector $x$, a set of weights $W$, a bias term $b$, and an activation function $\varphi$, the output of the hidden layer, $h$, is calculated as:

$$h = \varphi(Wx + b) \qquad\qquad (1)$$

Any number of activations functions may be used, including a sigmoid function, hyperbolic tangent function, rectified linear function, etc.

In some examples, shallow neural network spanned across each of the RNA module 202' and the imaging module 204' and the integration module 206', as well as, in some examples, additionally across the prediction module 208'.

In an example, the neural network implemented in the integration module 206' was trained using both RNA-seq features and image features generated from an image processing model of the imaging features module 204'. In the RNA module 202', the RNA-seq data was filtered using the LM22 gene list and the TPM values were log transformed (feature size = 547).

The image features included the mean and skewness values of intensity and texture features across all tiles in an image (feature size = 392). In a first layer of the neural network, each set of features was used as inputs to their own fully connected layer which used the rectified linear unit (ReLU) activation function.

$$\text{ReLU}(x) = \max\{0, x\} \tag{2}$$

A second layer, forming the integration module 206', concatenated outputs of the modularized dense layers to create an integrated set of features. The values from this second layer were then passed to an output layer that, in the illustrated example, formed the prediction module 206'.

FIG. 4 illustrates two pipelines for predicting relative immune proportion: a first pipeline 302 that operates on RNA sequencing data alone (referred to as the NN-RNA pipeline herein); and a second pipeline 304 that integrated RNA sequencing data with imaging feature data (reference to as the NN-RNA-image pipeline).

In an example embodiment, the prediction module 206' implements a Softmax function to predict the desired immune proportion. The Softmax function squashes an n-dimensional vector of real valued numbers into a new n-dimensional vector with values in the range (0,1] and the sum of all the values is equal to one. More specifically, given a set of values for Y = $\{y_1, y_2, ...y_n\}$

$$\text{Softmax}(y_k|Y) = \frac{e^{y_k}}{\sum_j e^{y_j}} \tag{3}$$

Since the model in this example was designed to predict a distribution, we trained it using the Kullback-Leibler divergence cost. The Kullback-Leibler divergence measures the divergence of a given probability distribution, Q, from a target probability distribution, P.

$$\text{KL}(P\|Q) = \sum_i P(i) \, log\frac{P(i)}{Q(i)} \tag{4}$$

To prevent over-fitting of our model, we applied an L2 regularization to the weights for every layer. This regularizes the model by diffusing the weight vectors so that the model uses all of its weights rather than relying on a subset of higher valued weights. We also sought to enforce the shallow neural network architecture by reducing layer sizes until performance degradation was observed. Our final cost function for training was:

$$C = \sum_i y_i \, log\frac{y_i}{\hat{y}_i} + \lambda \sum_L \|W^{(L)}\|_2 \tag{5}$$

Here, $y_i$ is the true value for the probability of the ith output, $\hat{y}_i$ is the predicted probability for the $i$th output, $\lambda$ is the L2 penalty coefficient, and $W^{(L)}$ are the weights for layer $L$.

The NN-RNA and NN-RNA-image pipelines 302, 304 were trained to predict either the distribution of different immune cell-types in the sample or the total fraction of the tumor immune infiltrate. These models were trained using the ADAM optimizer for batch gradient descent with a learning rate of 0.0005 and a $\lambda$ value of 0.01. For each result, the models were trained using leave one out cross validation and each model was trained until the test loss had not dropped for 30 epochs.

After training the models of the prediction module 208', we evaluated if we could apply transfer learning by using one model to boost the other. As shown in FIG. 6, for this, we used the outputs of an NN-Transfer pipeline 306, outputs determined for another instance of the prediction module, labeled contextual prediction module 208". The contextual prediction module 208", produces the total fraction of the tumor immune infiltrate, which was used as additional inputs to the second layer of the NN-RNA-image pipeline 304, that is, as additional inputs to the integration module 206', predicting the relative cell-type proportions. The NN-Transfer pipeline 306 was otherwise trained using the same methods and parameters described before. The modules 202", 204'', 206", and 208" are shown as separate for the NN-transfer pipeline 306 from their counterpart modules in the NN-RNA and NN-RNA-image pipelines 302 and 304. In other examples, the corresponding modules may be implemented by a single module for each process.

To examine the performance of the NN-RNA, NN-RNA-image, and NN-Transfer pipelines (302, 304, and 306, respectively), we tested the following four hypotheses. First, we tested whether the present techniques could effectively learn and predict immune infiltration cell-type proportions from RNA sequencing data alone, i.e., NN-RNA. Second, we tested whether integrating imaging features could further augment and improve infiltrate cell-type proportion prediction, i.e., NN-RNA-image. Third, we evaluated the flexibility of the present techniques by predicting the total fraction of tumor immune infiltrate instead of the proportion of five key immune cell-types, see, e.g., FIG. 6. Finally, we tested the hypothesis that integrating estimates of the total fraction of immune infiltrate could yet further augment and improve prediction of the key immune cell-types, i.e., NN-Transfer.

NN-RNA Example Comparison: Several groups have proposed methods for gene expression deconvolution using regression-based techniques. These include DeconRNASeq, which utilizes a non-negative linear regression approach, and a support vector regression (SVR)-based approach. We sought to determine if the present techniques could perform comparably to these algorithms when trained on RNA sequencing data only (NN-RNA). Due to commercial restrictions, we independently implemented a support vector regression deconvolution algorithm using the LM22 matrix, which is a previously published matrix of gene expression from different immune cells. Of the two regression-based techniques tested, we found that the SVR method performed better than DeconRNASeq, based on overall Pearson correlation.

To test the hypothesis that a neural network-based model (NN-RNA) could effectively learn immune cell proportions using RNA data only, we trained the system 100 on the RNA-seq data using expert pathologist scoring of infiltration and evaluated performance using leave-one-out cross validation. The NN-RNA architecture was used to predict relative proportions for B, CD4 T, CD8 T, MACs, and NK cells. To establish a baseline against SVR, the RNA-seq data was filtered using the genes in the LM22 matrix and the TPM values were log transformed.

We found that NN-RNA performed better than SVR based on overall Pearson correlation (FIG. 5A). We attribute this improvement to at least two factors: (1) whereas SVR is a linear deconvolution method, the present techniques are able to learn non-linear interactions between gene expression features; and (2) the present techniques are trained and tested using RNA sequencing data. Overall, we find that NN-RNA effectively learns immune cell-type proportions and demonstrates better accuracies than current methods.

NN-RNA-image Example Comparison: Information about infiltrating immune cells in histopathology slides is normally only accessible by overlaying additional multiplexed immunofluorescence or immunohistochemistry stains. We reasoned that embedded in microscopic H&E slides is latent information about the tumor-immune microenvironment, including the population structure and the underlying phenotypic states of the tumor and immune cells. Thus, we sought to test if integrating imaging features could further augment and improve the prediction of immune cell-type proportions.

To test this hypothesis, we obtained imaging features (i.e., visual texture and intensity features) from corresponding H&E images for each tumor sample. We utilized H&E image derived features due to the wide availability of H&E stained images used for cancer diagnosis and staging. To establish a baseline against NN-RNA, the RNA-seq data was filtered again using the LM22 gene list and the TPM values were log transformed. NN-RNA-image successfully predicted relative proportions for B, CD4 T, CD8 T, macrophage, and natural killer cells and was evaluated using leave-one-out cross validation.

As shown in FIG. 5a, NN-RNA-image boosted the prediction of immune cell-type proportions as accessed by overall Pearson correlation. Of note, improvements were preferentially observed for NK (R = .292 from .203), MACs (R = .569 from .508), and CD8 T cells (R=.408 from .233) (FIG. 5B). These results demonstrated that integration of imaging features can improve immune infiltrate cell-type prediction.

Predicting Total Tumor Immune Infiltration Fraction: The choice of using a shallow neural network-based architecture for NN-RNA-image in the example of FIG. 6 allowed us to demonstrate that the NN-RNA-image pipeline could be adopted for other related but distinct tasks. In the example of FIG. 6, we evaluated the flexibility NN-RNA-image in predicting the total fraction of the tumor immune infiltrate instead of the proportion of key immune cell-types.

The total immune fraction framework seeks to predict the abundance of immune cells in the overall tumor microenvironment, in contrast to relative proportions of immune subsets in the total leukocyte population (FIG. 4). In an implementation of FIG. 6, a pathologist was instructed to assess immune cells (leukocytes) based on cell morphologies from patient H&E slides. We implemented a version of NN-RNA-image to predict two outputs, percent immune and non-immune fractions. We trained NN-RNA-image using RNA-seq data filtered using the LM22 gene set and imaging features. We evaluated performance using leave-one-out cross validation.

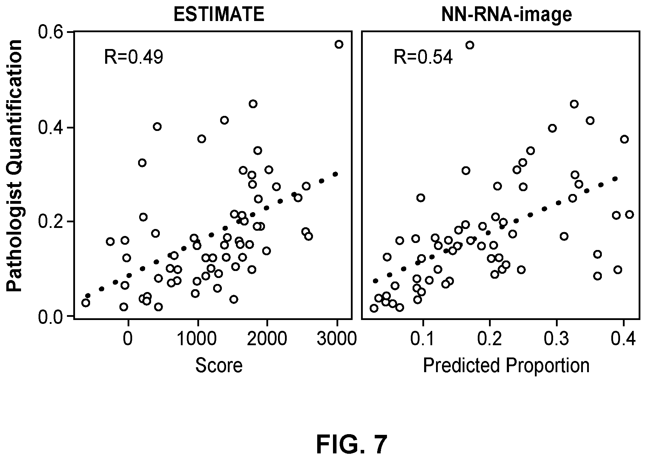

To benchmark our results, we analyzed samples with ESTIMATE, which is a tool for predicting tumor purity, and the presence of infiltrating stromal/immune cells in tumor tissues using gene expression data (FIG. 7). We found that a neural network-based model (NN-RNA-image) could be effectively adopted to learn the total immune infiltrate proportion. We found that our NN-RNA-image trained model performed better than ESTIMATE based on overall Pearson correlation. Taken together, NEXT provides a flexible framework for integrating RNA-seq and imaging features, and for predicting estimates of the tumor immune infiltrate.

NN-Transfer Example Comparison: With the pipeline estimating the total fraction of immune infiltrate using both RNA-seq and imaging features, we sought to test the fourth hypothesis that integrating estimates of the total fraction of immune infiltrate could further augment and improve the prediction of infiltration cell-type proportions. We reasoned that including the total immune and non-immune fraction may provide additional meaningful contextual features, for example. Concomitant predictions of the total fraction of immune infiltrate were concatenated to the RNA-seq and imaging feature representations in the first layer of the network. Consistent with previous models, the RNA-seq data was filtered using the LM22 gene list and the TPM values were log transformed. We trained this NN-Transfer pipeline model (FIG. 6) using RNA-seq and imaging features and evaluated performance using leave-one-out cross validation.

We found increased accuracy in immune infiltrate prediction as accessed by overall Pearson correlation (FIG. 5A, NN-Transfer). In this example, the increase in accuracy was driven largely by increased accuracy for specific immune cell-types, including B, CD4 T, and MACs (FIG. 5B). In contrast, there was minor decrease in accuracy for CD8 (.394 from .408) and NK (.279 from .292). In any event, as shown in FIGS. 5A and 5B, the present techniques exhibit flexibility and utility when including into the integration module additional contextual features as further laboratory-based modalities, suggesting that yet other relevant histological, molecular, and/or clinical features can be readily integrated into the present techniques.

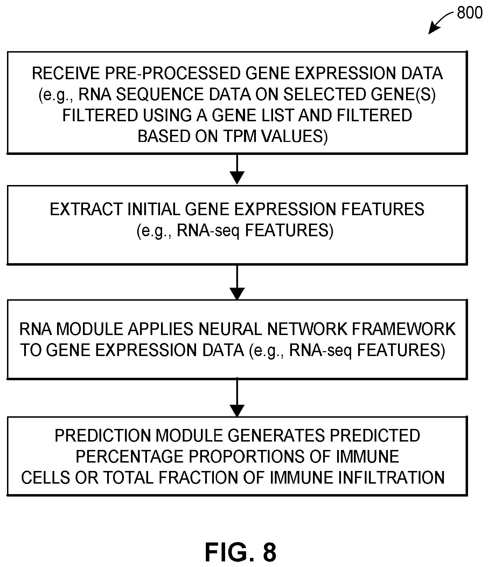

FIGS. 8 - 10 illustrate example processes for implementing methods herein, in accordance with some examples and as may be implemented by the immune infiltration predictor processing system 100.

In FIG. 8, a process 800 begins with receiving pre-processed gene expression data, such as RNA sequencing data on selected genes filtered using a gene list and filtered based on TMP values. Next, gene expression features are extracted from the received raw gene expression data, e.g., from the RNA sequencing data. An RNA module then applies the machine learning framework to the received gene expression features, e.g., by automatically transforming RNA sequencing data using one or more neural network layers. A prediction module then applies the outputs from the first layer (or layers) of the neural network to a prediction module formed of one or more neural network layers configured to predict immune cell percentages, which are then output as an immune infiltration score, indicating percentages of immune cells.

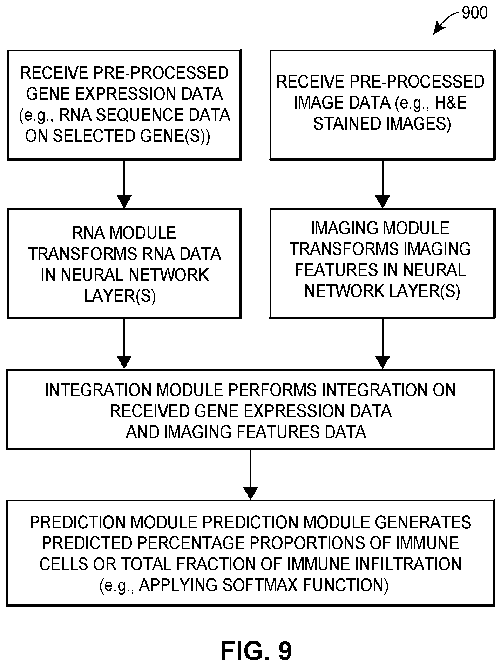

In FIG. 9, a process 900 begins with receiving pre-processed gene expression data, such as RNA sequencing data on selected genes filtered using a gene list and filtered based on TMP values, and pre-processed image data, such as stained histopathology images. In some examples, the pre-processing further includes extraction of gene expression features from received raw gene expression data, e.g., from the RNA sequencing data.

An RNA module applies the machine learning framework to the received pre-processed gene expression features, e.g., by automatically transforming RNA sequencing data using one or more neural network layers. An imaging module applies the machine learning framework to receive pre-processed image data, e.g., automatically transforming the image data using one or more neural network layers. The outputs from the RNA module and the imaging module are provided to an integration module of the machine learning framework and formed of one or more neural network layers and that integrates the received data and generates an integrated output of the gene expression data analysed with the imaging feature data (e.g., visual features and intensity features).

A prediction module then applies the outputs from a first layer (or layers) of the neural network to a prediction module formed of one or more neural network layers configured to predict immune cell percentages, which are then output as an immune infiltration score. In the illustrated example, the prediction module is configured to predict a percentage of specific immune cells or a total fraction of immune infiltration within a sample, expressed as a single overall percentage score.

In some examples, the RNA module, the imaging module, integration module, and prediction module are trained using an automated machine learning training process implemented on the immune infiltration predictor processing system. In some examples, these modules are trained using pathologist scored infiltration data.

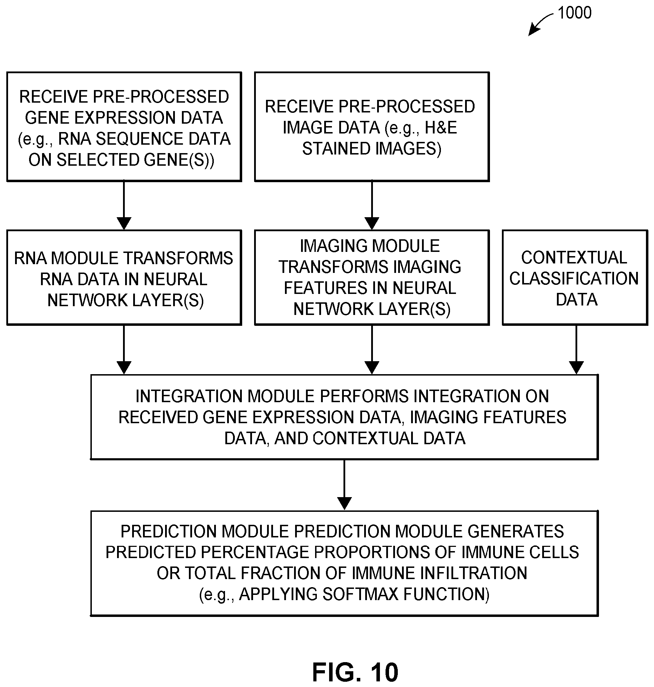

FIG. 10 illustrates a process 1000 similar to that of FIG. 9, with similar processes, except contextual classification data is also provided to the integration module for transforming gene expression, imaging features, and contextual data to provide an even more accurate classification output provided to the prediction module.

The framework of the neural network is flexible and the architecture can be easily expanded to incorporate additional information to further improve model performance. For instance, data from other assays like DNA sequencing, methylation profiling, immunofluorescence or other histological staining, flow or mass cytometry, and mass spectrometry can be integrated with the RNA and image components of the model in a similar fashion to that of the NN-Transfer (shown in FIG. 6). Additionally, the neural network framework can be applied to infer the composition for any biological mixture of cells. For instance, instead of immune cells, the same model can be adjusted to instead estimate the relative proportion of tumor and endothelial cells, which would provide information about how much vascularization is present in a tumor. The utility of these models is also not limited to cancer samples. Inferring the relative and absolute proportions of different immune cell types has value in many other disease areas, like lupus and rheumatoid arthritis, and is also useful in basic science research.

The present techniques present an important advancement in elaborating the tumor microenvironment and in predicting the immunological composition of individual patients. As detailed, we demonstrate that our framework is efficient and flexible, allowing investigators to integrate pre-existing routine clinical H&E stained slides with RNA-seq data. We also demonstrate increased accuracy in predicting the abundance of key immune cell subtypes in solid tumors when compared to expert pathologist assessment of IHC and conventional techniques.

The present techniques provide a neural network-based framework to predict immune infiltration proportions with models trained using gold standard expert pathologist reviewed IHC samples. We demonstrate a generalizable and flexible framework for clinical RNA-seq and imaging adapted for use broadening widespread pathological reporting of the immune infiltrate in tumor biopsies and guiding patient treatment decisions.

While the present techniques are described, in some examples, as used in shallow-level deep learning frameworks, we note that the present techniques are amenable to larger datasets because they allow for larger or more layers to increase learning capacity. Moreover, these techniques provide a principled approach for integrating other relevant molecular and clinical features from patients as new routine and widespread techniques become adopted.

Throughout this specification, plural instances may implement components, operations, or structures described as a single instance. Although individual operations of one or more methods are illustrated and described as separate operations, one or more of the individual operations may be performed concurrently, and nothing requires that the operations be performed in the order illustrated. Structures and functionality presented as separate components in example configurations may be implemented as a combined structure or component. Similarly, structures and functionality presented as a single component may be implemented as separate components or multiple components. These and other variations, modifications, additions, and improvements fall within the scope of the subject matter herein.

Additionally, certain embodiments are described herein as including logic or a number of routines, subroutines, applications, or instructions. These may constitute either software (e.g., code embodied on a machine-readable medium or in a transmission signal) or hardware. In hardware, the routines, etc., are tangible units capable of performing certain operations and may be configured or arranged in a certain manner. In example embodiments, one or more computer systems (e.g., a standalone, client or server computer system) or one or more hardware modules of a computer system (e.g., a processor or a group of processors) may be configured by software (e.g., an application or application portion) as a hardware module that operates to perform certain operations as described herein.

In various embodiments, a hardware module may be implemented mechanically or electronically. For example, a hardware module may comprise dedicated circuitry or logic that is permanently configured (e.g., as a special-purpose processor, such as a microcontroller, field programmable gate array (FPGA) or an application-specific integrated circuit (ASIC)) to perform certain operations. A hardware module may also comprise programmable logic or circuitry (e.g., as encompassed within a processor, central processing unit, graphic processing unit, or other programmable processor) that is temporarily configured by software to perform certain operations. It will be appreciated that the decision to implement a hardware module mechanically, in dedicated and permanently configured circuitry, or in temporarily configured circuitry (e.g., configured by software) may be driven by cost and time considerations.

Accordingly, the term "hardware module" should be understood to encompass a tangible entity, be that an entity that is physically constructed, permanently configured (e.g., hardwired), or temporarily configured (e.g., programmed) to operate in a certain manner or to perform certain operations described herein. Considering embodiments in which hardware modules are temporarily configured (e.g., programmed), each of the hardware modules need not be configured or instantiated at any one instance in time. For example, where the hardware modules comprise a processor configured using software, the processor may be configured as respective different hardware modules at different times. Software may accordingly configure a processor, for example, to constitute a particular hardware module at one instance of time and to constitute a different hardware module at a different instance of time.

Hardware modules can provide information to, and receive information from, other hardware modules. Accordingly, the described hardware modules may be regarded as being communicatively coupled. Where multiple of such hardware modules exist contemporaneously, communications may be achieved through signal transmission (e.g., over appropriate circuits and buses) that connects the hardware modules. In embodiments in which multiple hardware modules are configured or instantiated at different times, communications between such hardware modules may be achieved, for example, through the storage and retrieval of information in memory structures to which the multiple hardware modules have access. For example, one hardware module may perform an operation and store the output of that operation in a memory device to which it is communicatively coupled. A further hardware module may then, at a later time, access the memory device to retrieve and process the stored output. Hardware modules may also initiate communications with input or output devices, and can operate on a resource (e.g., a collection of information).

The various operations of the example methods described herein can be performed, at least partially, by one or more processors that are temporarily configured (e.g., by software) or permanently configured to perform the relevant operations. Whether temporarily or permanently configured, such processors may constitute processor-implemented modules that operate to perform one or more operations or functions. The modules referred to herein may, in some example embodiments, comprise processor-implemented modules.

Similarly, the methods or routines described herein may be at least partially processor-implemented. For example, at least some of the operations of a method can be performed by one or more processors or processor-implemented hardware modules. The performance of certain of the operations may be distributed among the one or more processors, not only residing within a single machine, but also deployed across a number of machines. In some example embodiments, the processor or processors may be located in a single location (e.g., within a home environment, an office environment or as a server farm), while in other embodiments the processors may be distributed across a number of locations.

The performance of certain of the operations may be distributed among the one or more processors, not only residing within a single machine, but also deployed across a number of machines. In some example embodiments, the one or more processors or processor-implemented modules may be located in a single geographic location (e.g., within a home environment, an office environment, or a server farm). In other example embodiments, the one or more processors or processor-implemented modules may be distributed across a number of geographic locations.

Unless specifically stated otherwise, discussions herein using words such as "processing," "computing," "calculating," "determining," "presenting," "displaying," or the like may refer to actions or processes of a machine (e.g., a computer) that manipulates or transforms data represented as physical (e.g., electronic, magnetic, or optical) quantities within one or more memories (e.g., volatile memory, non-volatile memory, or a combination thereof), registers, or other machine components that receive, store, transmit, or display information.

As used herein any reference to "one embodiment" or "an embodiment" means that a particular element, feature, structure, or characteristic described in connection with the embodiment is included in at least one embodiment. The appearances of the phrase "in one embodiment" in various places in the specification are not necessarily all referring to the same embodiment.

Some embodiments may be described using the expression "coupled" and "connected" along with their derivatives. For example, some embodiments may be described using the term "coupled" to indicate that two or more elements are in direct physical or electrical contact. The term "coupled," however, may also mean that two or more elements are not in direct contact with each other, but yet still co-operate or interact with each other. The embodiments are not limited in this context.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

In addition, use of the "a" or "an" are employed to describe elements and components of the embodiments herein. This is done merely for convenience and to give a general sense of the description. This description, and the claims that follow, should be read to include one or at least one and the singular also includes the plural unless it is obvious that it is meant otherwise.

This detailed description is to be construed as an example only and does not describe every possible embodiment, as describing every possible embodiment would be impractical, if not impossible. One could implement numerous alternate embodiments, using either current technology or technology developed after the filing date of this application.

**Claims**

1. A computing device configured to generate an immune infiltration prediction score, the computing device comprising one or more processors configured to:

   obtain gene expression data from one or more gene expression datasets with the gene expression data corresponding to one or more primary tumor tissue samples, where the gene expression data reflects the molecular characteristics of the tumor;
   obtain a set of stained histopathology images from one or more image sources including hematoxylin and eosin (H&E) staining for structural visualization corresponding to the one or more tissue samples and indicating a visual representation of the tumor's structural and immune characteristics;
   determine imaging features from the set of stained histopathology images, the imaging features comprising texture and/or intensity features indicating the tumor's structural and immune landscape;
   within a neural network framework, process the obtained gene expression data through a gene expression neural network layer(s) configured to perform molecular data analysis and concurrently, process the determined imaging features through an imaging feature neural network layer(s) configured to extract structural and immune-related visual information;
   integrate, within the neural network framework, the outputs of both the gene expression neural network layer(s) and the imaging feature neural network layer(s) to produce an integrated neural network output that encapsulates both the molecular and visual characteristics of the tumor; and

apply a prediction function to the integrated neural network output to derive an immune infiltration score for the one or more tissue samples, wherein the score indicates the immune cell composition within the tumor.

2. A computing device configured to generate an immune infiltration prediction score, the computing device comprising one or more processors configured to:

obtain gene expression data from one or more gene expression datasets with the gene expression data corresponding to one or more primary tumor tissue samples, where the gene expression data reflects the molecular characteristics of the tumor;

obtain a set of stained histopathology images from one or more image sources including images from hematoxylin and eosin (H&E) staining for structural visualization corresponding to the one or more tissue samples and indicating a visual representation of the tumor's structural and immune characteristics;

determine imaging features from the set of stained histopathology images, the imaging features comprising texture and/or intensity features indicating the tumor's structural and immune landscape;

obtain contextual data corresponding to the one or more tissue samples;

within a neural network framework, process the obtained gene expression data through a gene expression neural network layer(s) configured to perform molecular data analysis and concurrently, (i) process the determined imaging features through an imaging feature neural network layer(s) configured to extract structural and immune-related visual information, and (ii) process the contextual data through contextual feature neural network layer(s) configured to incorporate contextual aspects of the tissue samples;

integrate, within the neural network framework, the outputs of the gene expression neural network layer(s), the imaging feature neural network layer(s), and the contextual feature neural network layer(s) to produce an integrated neural network output that encapsulates the molecular, visual and contextual characteristics of the tumor; and

apply a prediction function to the integrated neural network output to derive an immune infiltration score for the one or more tissue samples, wherein the score indicates the immune cell composition within the tumor.

3. The computing device of claim 1 or 2, wherein the gene expression data is RNA sequencing data.

4. The computing device of claim 1 or 2, wherein the neural network framework comprises two neural network layers.

5. The computing device of claim 1 or 2, wherein the imaging features comprise mean, standard deviation, skewness, and/or sum of image gray level, image red, green, blue layers, stain layers, optical density, hue, and/or saturation.

6. The computing device of claim 1 or 2, wherein the imaging features comprise Zernike moments, threshold adjacency analysis values, local binary patterns, gray scale co-occurrence matrix, and/or difference of Gaussian statistical measures.

7. The computing device of claim 1 or 2, wherein the prediction function is Softmax function.

8. The computing device of claim 1 or 2, wherein immune infiltration score comprises a predicted percentage of natural killer (NK) cells, (MAC) macrophage cells, CD4 T cells, CD8 T cells, and B cells, regulatory T cells, Dendritic cells, monocytes, Mast cells, Eosinophils, and Neutrophils.

9. The computing device of claim 1 or 2, wherein immune infiltration score comprises a predicted percentage of others cells, including stromal cells, vasculature cells, fat cells, tumor cells, stem cells, neural cells, progenitor cells, innate lymphoid cells, microglial cells, leukocytes, naive B cells, memory B cells, Plasma cells, CD8 T cells, naive CD4 T cells, memory CD4 T cells, follicular helper T cells, regulatory T cells, gamma delta T cells, Th17 T cells, unstimulated NK cells, stimulated NK cells, Macrophages MO, Macrophages M1, Macrophages M2, unstimulated Dendritic cells, stimulated Dendritic cells, unstimulated Mast cells, stimulated Mast cells.

10. The computing device of claim 1 or 2, wherein the contextual data is a total immune fraction or total tumor fraction.

11. A computer-implemented method to generate an immune infiltration prediction score, the method comprising:

obtaining gene expression data from one or more gene expression datasets with the gene expression data corresponding to one or more primary tumor tissue samples, where the gene expression data reflects the molecular characteristics of the tumor;

obtaining a set of stained histopathology images from one or more image sources including hematoxylin and eosin (H&E) staining for structural visualization corresponding to the one or more tissue samples and indicating a visual representation of the tumor's structural and immune characteristics;

determining imaging features from the set of stained histopathology images, the imaging features comprising texture and/or intensity features indicating the tumor's structural and immune landscape;

within a neural network framework, processing the obtained gene expression data through a gene expression neural network layer(s) configured to perform molecular data analysis and concurrently, processing the determined imaging features through an imaging feature neural network layer(s) configured to extract structural and immune-related visual information;

integrating, within the neural network framework, the outputs of both the gene expression neural network layer(s) and the imaging feature neural network layer(s) to produce an integrated neural network output that encapsulates both the molecular and visual characteristics of the tumor; and

applying a prediction function to the integrated neural network output and outputting an immune infiltration score for the one or more tissue samples, wherein the score indicates the immune cell composition within the tumor.

**12.** A computer-implemented method to generate an immune infiltration prediction score, the method comprising:

obtaining gene expression data from one or more gene expression datasets with the gene expression data corresponding to one or more primary tumor tissue samples, where the gene expression data reflects the molecular characteristics of the tumor;

obtaining a set of stained histopathology images from one or more image sources including hematoxylin and eosin (H&E) staining for structural visualization corresponding to the one or more tissue samples and indicating a visual representation of the tumor's structural and immune characteristics;

determining imaging features from the set of stained histopathology images, the imaging features comprising texture and/or intensity features indicating the tumor's structural and immune landscape;

obtaining contextual data corresponding to the one or more tissue samples;

within a neural network framework, processing the obtained the gene expression data through a gene expression neural network layer(s) configured to perform molecular data analysis and concurrently, (i) processing the determined imaging features through an imaging feature neural network layer(s) configured to extract structural and immune-related visual information, and (ii) processing the contextual data through contextual feature neural network layer(s) configured to incorporate contextual aspects of the tissue samples;

integrating, within the neural network framework, the outputs of the gene expression neural network layer(s), the imaging feature neural network layer(s), and the contextual feature neural network layer(s) to produce an integrated neural network output that encapsulates the molecular, visual and contextual characteristics of the tumor; and

applying a prediction function to the integrated neural network output to derive an immune infiltration score for the one or more tissue samples, wherein the score indicates the immune cell composition within the tumor.

**Patentansprüche**

**1.** Rechenvorrichtung, die konfiguriert ist, um einen Immuninfiltrations-Vorhersagewert zu erzeugen, wobei die Rechenvorrichtung einen oder mehrere Prozessoren umfasst, die konfiguriert sind, um:

Genexpressionsdaten aus einem oder mehreren Genexpressionsdatensätzen zu erhalten, wobei die Genexpressionsdaten einer oder mehreren primären Tumorgewebeproben entsprechen, wobei die Genexpressionsdaten die molekularen Eigenschaften des Tumors widerspiegeln;

einen Satz gefärbter histopathologischer Bilder aus einer oder mehreren Bildquellen zu erhalten, einschließlich Hämatoxylin- und Eosin (H&E)-Färbung zur strukturellen Visualisierung, die der einen oder den mehreren Gewebeproben entspricht und eine visuelle Darstellung der strukturellen und Immuneigenschaften des Tumors anzeigt;

Bildgebungsmerkmale aus dem Satz gefärbter histopathologischer Bilder zu bestimmen, wobei die Bildgebungsmerkmale Textur- und/oder Intensitätsmerkmale umfassen, die die strukturelle und Immunlandschaft des Tumors anzeigen;

innerhalb eines neuronalen Netzwerk-Frameworks die erhaltenen Genexpressionsdaten durch neuronale Genexpressionsnetzwerkschicht(en) zu verarbeiten, die konfiguriert sind, um eine molekulare Datenanalyse durchzuführen und gleichzeitig die bestimmten Bildgebungsmerkmale durch neuronale Bildgebungsmerkmalsnetzwerkschicht(en) zu verarbeiten, die konfiguriert sind, um strukturelle und immunbezogene visuelle Informationen zu extrahieren;

innerhalb des neuronalen Netzwerk-Frameworks die Ausgaben sowohl der neuronalen Genexpressionsnetzwerkschicht(en) als auch der neuronalen Bildgebungsmerkmalsnetzwerkschicht(en) zu integrieren, um eine integrierte neuronale Netzwerkausgabe zu erzeugen, die sowohl die molekularen als auch die visuellen Eigenschaften des Tumors einkapselt; und

eine Vorhersagefunktion auf die integrierte neuronale Netzwerkausgabe anzuwenden, um einen Immuninfiltrationswert für die eine oder die mehreren Gewebeproben abzuleiten, wobei der Wert die Immunzellzusammensetzung innerhalb des Tumors anzeigt.

2. Rechenvorrichtung, die konfiguriert ist, um einen Immuninfiltrations-Vorhersagewert zu erzeugen, wobei die Rechenvorrichtung einen oder mehrere Prozessoren umfasst, die konfiguriert sind, um:

Genexpressionsdaten aus einem oder mehreren Genexpressionsdatensätzen zu erhalten, wobei die Genexpressionsdaten einer oder mehreren primären Tumorgewebeproben entsprechen, wobei die Genexpressionsdaten die molekularen Eigenschaften des Tumors widerspiegeln;

einen Satz gefärbter histopathologischer Bilder aus einer oder mehreren Bildquellen zu erhalten, einschließlich Bildern aus Hämatoxylin- und Eosin (H&E)-Färbung zur strukturellen Visualisierung, die der einen oder den mehreren Gewebeproben entspricht und eine visuelle Darstellung der strukturellen und Immuneigenschaften des Tumors anzeigt;

Bildgebungsmerkmale aus dem Satz gefärbter histopathologischer Bilder zu bestimmen, wobei die Bildgebungsmerkmale Textur- und/oder Intensitätsmerkmale umfassen, die die strukturelle und Immunlandschaft des Tumors anzeigen;

Kontextdaten zu erhalten, die der einen oder den mehreren Gewebeproben entsprechen;

innerhalb eines neuronalen Netzwerk-Frameworks die erhaltenen Genexpressionsdaten durch neuronale Genexpressionsnetzwerkschicht(en) zu verarbeiten, die konfiguriert sind, um eine molekulare Datenanalyse durchzuführen und gleichzeitig (i) die bestimmten Bildgebungsmerkmale durch neuronale Bildgebungsmerkmalsnetzwerkschicht(en) zu verarbeiten, die konfiguriert sind, um strukturelle und immunbezogene visuelle Informationen zu extrahieren, und (ii) die Kontextdaten durch neuronale Kontextmerkmalsnetzwerkschicht(en) zu verarbeiten, die konfiguriert sind, um Kontextaspekte der Gewebeproben zu integrieren;

innerhalb des neuronalen Netzwerk-Frameworks die Ausgaben der neuronalen Genexpressionsnetzwerkschicht(en), der neuronalen Bildgebungsmerkmalsnetzwerkschicht(en) und der neuronalen Kontextmerkmalsnetzwerkschicht(en) zu integrieren, um eine integrierte neuronale Netzwerkausgabe zu erzeugen, die die molekularen, visuellen und Kontexteigenschaften des Tumors einkapselt; und

eine Vorhersagefunktion auf die integrierte neuronale Netzwerkausgabe anzuwenden, um einen Immuninfiltrationswert für die eine oder die mehreren Gewebeproben abzuleiten, wobei der Wert die Immunzellzusammensetzung innerhalb des Tumors anzeigt.

3. Rechenvorrichtung nach Anspruch 1 oder 2, wobei die Genexpressionsdaten RNA-Sequenzierungsdaten sind.

4. Rechenvorrichtung nach Anspruch 1 oder 2, wobei das neuronale Netzwerk-Framework zwei neuronale Netzwerkschichten umfasst.

5. Rechenvorrichtung nach Anspruch 1 oder 2, wobei die Bildgebungsmerkmale Mittelwert, Standardabweichung, Schiefe und/oder Summe von Bildgraustufe, Bild-Rot-, Grün-, Blauschichten, Färbeschichten, optischer Dichte, Farbton und/oder Sättigung umfassen.

6. Rechenvorrichtung nach Anspruch 1 oder 2, wobei die Bildgebungsmerkmale Zernike-Momente, Schwellenadjazenzanalysewerte, lokale Binärmuster, Graustufen-Koauftrittsmatrix und/oder Differenz von Gauß'schen statistischen Maßen umfassen.

7. Rechenvorrichtung nach Anspruch 1 oder 2, wobei die Vorhersagefunktion Softmax-Funktion ist.

8. Rechenvorrichtung nach Anspruch 1 oder 2, wobei der Immuninfiltrationswert einen vorhergesagten Prozentsatz von natürlichen Killerzellen (NK-Zellen), Makrophagenzellen (MAC-Zellen), CD4-T-Zellen, CD8-T-Zellen und B-Zellen, regulatorischen T-Zellen, dendritischen Zellen, Monozyten, Mastzellen, Eosinophilen und Neutrophilen umfasst.

9. Rechenvorrichtung nach Anspruch 1 oder 2, wobei der Immuninfiltrationswert einen vorhergesagten Prozentsatz von anderen Zellen umfasst, einschließlich Stromazellen, Gefäßzellen, Fettzellen, Tumorzellen, Stammzellen, neuronalen Zellen, Vorläuferzellen, angeborenen Lymphoidzellen, Mikrogliazellen, Leukozyten, naiven B-Zellen, Gedächtnis-B-Zellen, Plasmazellen, CD8-T-Zellen, naiven CD4-T-Zellen, Gedächtnis-CD4-T-Zellen, follikulären Helfer-T-Zellen, regulatorischen T-Zellen, Gamma-Delta-T-Zellen, Th17-T-Zellen, unstimulierten NK-Zellen, stimulierten NK-Zellen, Makrophagen MO, Makrophagen M1, Makrophagen M2, unstimulierten dendritischen Zellen, stimulierten dendritischen Zellen, unstimulierten Mastzellen, stimulierten Mastzellen.

10. Rechenvorrichtung nach Anspruch 1 oder 2, wobei die Kontextdaten eine Gesamtimmunfraktion oder eine Gesamttumorfraktion sind.

11. Computerimplementiertes Verfahren zum Erzeugen eines Immuninfiltrations-Vorhersagewerts, wobei das Verfahren Folgendes umfasst:

Erhalten von Genexpressionsdaten aus einem oder mehreren Genexpressionsdatensätzen, wobei die Genexpressionsdaten einer oder mehreren primären Tumorgewebeproben entsprechen, wobei die Genexpressionsdaten die molekularen Eigenschaften des Tumors widerspiegeln;

Erhalten eines Satzes gefärbter histopathologischer Bilder aus einer oder mehreren Bildquellen, einschließlich Hämatoxylin- und Eosin (H&E)-Färbung zur strukturellen Visualisierung, die der einen oder den mehreren Gewebeproben entspricht und eine visuelle Darstellung der strukturellen und Immuneigenschaften des Tumors anzeigt;

Bestimmen von Bildgebungsmerkmalen aus dem Satz gefärbter histopathologischer Bilder, wobei die Bildgebungsmerkmale Textur- und/oder Intensitätsmerkmale umfassen, die die strukturelle und Immunlandschaft des Tumors anzeigen;

innerhalb eines neuronalen Netzwerk-Frameworks Verarbeiten der erhaltenen Genexpressionsdaten durch neuronale Genexpressionsnetzwerkschicht(en), die konfiguriert sind, um eine molekulare Datenanalyse durchzuführen und gleichzeitig die bestimmten Bildgebungsmerkmale durch neuronale Bildgebungsmerkmalsnetzwerkschicht(en) zu verarbeiten, die konfiguriert sind, um strukturelle und immunbezogene visuelle Informationen zu extrahieren;

innerhalb des neuronalen Netzwerk-Frameworks Integrieren der Ausgaben sowohl der neuronalen Genexpressionsnetzwerkschicht(en) als auch der neuronalen Bildgebungsmerkmalsnetzwerkschicht(en), um eine integrierte neuronale Netzwerkausgabe zu erzeugen, die sowohl die molekularen als auch die visuellen Eigenschaften des Tumors einkapselt; und

Anwenden einer Vorhersagefunktion auf die integrierte neuronale Netzwerkausgabe und Ausgeben eines Immuninfiltrationswerts für die eine oder die mehreren Gewebeproben, wobei der Wert die Immunzellzusammensetzung innerhalb des Tumors anzeigt.

12. Computerimplementiertes Verfahren zum Erzeugen eines Immuninfiltrations-Vorhersagewerts, wobei das Verfahren Folgendes umfasst:

Erhalten von Genexpressionsdaten aus einem oder mehreren Genexpressionsdatensätzen, wobei die Genexpressionsdaten einer oder mehreren primären Tumorgewebeproben entsprechen, wobei die Genexpressionsdaten die molekularen Eigenschaften des Tumors widerspiegeln;

Erhalten eines Satzes gefärbter histopathologischer Bilder aus einer oder mehreren Bildquellen, einschließlich Hämatoxylin- und Eosin (H&E)-Färbung zur strukturellen Visualisierung, die der einen oder den mehreren Gewebeproben entspricht und eine visuelle Darstellung der strukturellen und Immuneigenschaften des Tumors anzeigt;

Bestimmen von Bildgebungsmerkmalen aus dem Satz gefärbter histopathologischer Bilder, wobei die Bildgebungsmerkmale Textur- und/oder Intensitätsmerkmale umfassen, die die strukturelle und Immunlandschaft des Tumors anzeigen;

Erhalten von Kontextdaten, die der einen oder den mehreren Gewebeproben entsprechen;

innerhalb eines neuronalen Netzwerk-Frameworks Verarbeiten der erhaltenen Genexpressionsdaten durch neuronale Genexpressionsnetzwerkschicht(en), die konfiguriert sind, um eine molekulare Datenanalyse durchzuführen und gleichzeitig (i) die bestimmten Bildgebungsmerkmale durch neuronale Bildgebungsmerkmalsnetzwerkschicht(en) zu verarbeiten, die konfiguriert sind, um strukturelle und immunbezogene visuelle Informationen zu extrahieren, und (ii) die Kontextdaten durch neuronale Kontextmerkmalsnetzwerkschicht(en) zu verarbeiten, die konfiguriert sind, um Kontextaspekte der Gewebeproben zu integrieren;

innerhalb des neuronalen Netzwerk-Frameworks die Ausgaben der neuronalen Genexpressionsnetzwerkschicht(en), der neuronalen Bildgebungsmerkmalsnetzwerkschicht(en) und der neuronalen Kontextmerkmalsnetzwerkschicht(en) zu integrieren, um eine integrierte neuronale Netzwerkausgabe zu erzeugen, die die molekularen, visuellen und Kontexteigenschaften des Tumors einkapselt; und

Anwenden einer Vorhersagefunktion auf die integrierte neuronale Netzwerkausgabe, um einen Immuninfiltrationswert für die eine oder die mehreren Gewebeproben abzuleiten, wobei der Wert die Immunzellzusammensetzung innerhalb des Tumors anzeigt.

**Revendications**

1. Dispositif informatique configuré pour générer un score de prédiction d'infiltration immunitaire, le dispositif informatique comprenant un ou plusieurs processeurs configurés pour :

obtenir des données d'expression génique à partir d'un ou plusieurs ensembles de données d'expression génique avec les données d'expression génique correspondant à un ou plusieurs échantillons de tissu tumoral primaire, où les données d'expression génique reflètent les caractéristiques moléculaires de la tumeur ;

obtenir un ensemble d'images histopathologiques colorées à partir d'une ou plusieurs sources d'image comprenant une coloration à l'hématoxyline et l'éosine (H&E) pour une visualisation structurelle correspondant aux un ou plusieurs échantillons de tissu et indiquant une représentation visuelle des caractéristiques structurelles et immunitaires de la tumeur ;

déterminer des caractéristiques d'imagerie à partir de l'ensemble d'images histopathologiques colorées, les caractéristiques d'imagerie comprenant des caractéristiques de texture et/ou d'intensité indiquant le paysage structurel et immunitaire de la tumeur ;

dans une infrastructure de réseau neuronal, traiter les données d'expression génique obtenues par l'intermédiaire d'une ou plusieurs couches de réseau neuronal d'expression génique configurées pour effectuer une analyse de données moléculaires et traiter simultanément les caractéristiques d'imagerie déterminées par l'intermédiaire d'une ou plusieurs couches de réseau neuronal de caractéristiques d'imagerie configurées pour extraire des informations visuelles structurelles et immunitaires ;

intégrer, dans l'infrastructure de réseau neuronal, les sorties à la fois de la ou des couches de réseau neuronal d'expression génique et de la ou des couches de réseau neuronal de caractéristiques d'imagerie pour produire une sortie de réseau neuronal intégrée qui encapsule à la fois les caractéristiques moléculaires et visuelles de la tumeur ; et

appliquer une fonction de prédiction à la sortie de réseau neuronal intégrée pour dériver un score d'infiltration immunitaire pour les un ou plusieurs échantillons de tissu, dans lequel le score indique la composition de cellules immunitaires dans la tumeur.

2. Dispositif informatique configuré pour générer un score de prédiction d'infiltration immunitaire, le dispositif informatique comprenant un ou plusieurs processeurs configurés pour :

obtenir des données d'expression génique à partir d'un ou plusieurs ensembles de données d'expression génique avec les données d'expression génique correspondant à un ou plusieurs échantillons de tissu tumoral primaire, où les données d'expression génique reflètent les caractéristiques moléculaires de la tumeur ;

obtenir un ensemble d'images histopathologiques colorées à partir d'une ou plusieurs sources d'image comprenant des images provenant d'une coloration à l'hématoxyline et l'éosine (H&E) pour une visualisation structurelle correspondant aux un ou plusieurs échantillons de tissu et indiquant une représentation visuelle des caractéristiques structurelles et immunitaires de la tumeur ;

déterminer des caractéristiques d'imagerie à partir de l'ensemble d'images histopathologiques colorées, les caractéristiques d'imagerie comprenant des caractéristiques de texture et/ou d'intensité indiquant le paysage structurel et immunitaire de la tumeur ;

obtenir des données contextuelles correspondant aux un ou plusieurs échantillons de tissu ;

dans une infrastructure de réseau neuronal, traiter les données d'expression génique obtenues par l'intermédiaire d'une ou plusieurs couches de réseau neuronal d'expression génique configurées pour effectuer une analyse de données moléculaires et traiter simultanément, (i) les caractéristiques d'imagerie déterminées par l'intermédiaire d'une ou plusieurs couches de réseau neuronal de caractéristiques d'imagerie configurées pour extraire des informations visuelles structurelles et immunitaires, et (ii) les données contextuelles par l'intermédiaire d'une ou plusieurs couches de réseau neuronal de caractéristiques contextuelles configurées pour incorporer des aspects contextuels des échantillons de tissu ;

intégrer, dans l'infrastructure de réseau neuronal, les sorties de la ou des couches de réseau neuronal d'expression génique, de la ou des couches de réseau neuronal de caractéristiques d'imagerie, et de la ou des couches de réseau neuronal de caractéristiques contextuelles pour produire une sortie de réseau neuronal intégré qui encapsule les caractéristiques moléculaires, visuelles et contextuelles de la tumeur ; et

appliquer une fonction de prédiction à la sortie de réseau neuronal intégré pour dériver un score d'infiltration immunitaire pour les un ou plusieurs échantillons de tissu, dans lequel le score indique la composition de cellules immunitaires dans la tumeur.

3. Dispositif informatique selon la revendication 1 ou 2, dans lequel les données d'expression génique sont des données de séquençage d'ARN.

4. Dispositif informatique selon la revendication 1 ou 2, dans lequel l'infrastructure de réseau neuronal comprend deux couches de réseau neuronal.

5. Dispositif informatique selon la revendication 1 ou 2, dans lequel les caractéristiques d'imagerie comprennent la moyenne, l'écart type, l'asymétrie et/ou la somme du niveau de gris d'image, des couches rouge, verte, bleue d'image, des couches de coloration, la densité optique, la teinte et/ou la saturation.

6. Dispositif informatique selon la revendication 1 ou 2, dans lequel les caractéristiques d'imagerie comprennent des moments de Zernike, des valeurs d'analyse d'adjacence de seuil, des motifs binaires locaux, une matrice de co-occurrence d'échelle de gris et/ou une différence de mesures statistiques gaussiennes.

7. Dispositif informatique selon la revendication 1 ou 2, dans lequel la fonction de prédiction est la fonction Softmax.

8. Dispositif informatique selon la revendication 1 ou 2, dans lequel le score d'infiltration immunitaire comprend un pourcentage prédit de cellules tueuses naturelles (NK), de cellules macrophages (MAC), de cellules T CD4, de cellules T CD8 et de cellules B, de cellules T régulatrices, de cellules dendritiques, de monocytes, de mastocytes, d'éosinophiles et de neutrophiles.

9. Dispositif informatique selon la revendication 1 ou 2, dans lequel le score d'infiltration immunitaire comprend un pourcentage prédit d'autres cellules, y compris des cellules stromales, des cellules vasculaires, des cellules adipeuses, des cellules tumorales, des cellules souches, des cellules neuronales, des cellules progénitrices, des cellules lymphoïdes innées, des cellules microgliales, des leucocytes, des cellules B naïves, des cellules B à mémoire, des cellules plasmatiques, des cellules T CD8, des cellules T CD4 naïves, des cellules T CD4 à mémoire, des cellules T auxiliaires folliculaires, des cellules T régulatrices, des cellules T gamma delta, des cellules T Th17, des cellules NK non stimulées, des cellules NK stimulées, des macrophages MO, des macrophages M1, des macrophages M2, des cellules dendritiques non stimulées, des cellules dendritiques stimulées, des mastocytes non stimulés et des mastocytes stimulés.

10. Dispositif informatique selon la revendication 1 ou 2, dans lequel les données contextuelles sont une fraction immunitaire totale ou une fraction tumorale totale.

11. Procédé mis en œuvre par ordinateur pour générer un score de prédiction d'infiltration immunitaire, le procédé comprenant :

l'obtention de données d'expression génique à partir d'un ou plusieurs ensembles de données d'expression génique avec les données d'expression génique correspondant à un ou plusieurs échantillons de tissu tumoral primaire, où les données d'expression génique reflètent les caractéristiques moléculaires de la tumeur ;

l'obtention d'un ensemble d'images histopathologiques colorées à partir d'une ou plusieurs sources d'image comprenant une coloration à l'hématoxyline et l'éosine (H&E) pour une visualisation structurelle correspondant aux un ou plusieurs échantillons de tissu et indiquant une représentation visuelle des caractéristiques structurelles et immunitaires de la tumeur ;

la détermination de caractéristiques d'imagerie à partir de l'ensemble d'images histopathologiques colorées, les caractéristiques d'imagerie comprenant des caractéristiques de texture et/ou d'intensité indiquant le paysage structurel et immunitaire de la tumeur ;

dans une infrastructure de réseau neuronal, le traitement des données d'expression génique obtenues par l'intermédiaire d'une ou plusieurs couches de réseau neuronal d'expression génique configurées pour effectuer une analyse de données moléculaires et traiter simultanément les caractéristiques d'imagerie déterminées par l'intermédiaire d'une ou plusieurs couches de réseau neuronal de caractéristiques d'imagerie configurées pour extraire des informations visuelles structurelles et immunitaires ;

l'intégration, dans l'infrastructure de réseau neuronal, des sorties à la fois de la ou des couches de réseau neuronal d'expression génique et de la ou des couches de réseau neuronal de caractéristiques d'imagerie pour produire une sortie de réseau neuronal intégrée qui encapsule à la fois les caractéristiques moléculaires et visuelles de la tumeur ; et

l'application d'une fonction de prédiction à la sortie de réseau neuronal intégrée et la sortie d'un score d'infiltration immunitaire pour les un ou plusieurs échantillons de tissu, dans lequel le score indique la composition de cellules immunitaires dans la tumeur.

12. Procédé mis en œuvre par ordinateur pour générer un score de prédiction d'infiltration immunitaire, le procédé comprenant :

l'obtention de données d'expression génique à partir d'un ou plusieurs ensembles de données d'expression génique avec les données d'expression génique correspondant à un ou plusieurs échantillons de tissu tumoral primaire, où les données d'expression génique reflètent les caractéristiques moléculaires de la tumeur ;

l'obtention d'un ensemble d'images histopathologiques colorées à partir d'une ou plusieurs sources d'image comprenant une coloration à l'hématoxyline et l'éosine (H&E) pour une visualisation structurelle correspondant aux un ou plusieurs échantillons de tissu et indiquant une représentation visuelle des caractéristiques structurelles et immunitaires de la tumeur ;

la détermination de caractéristiques d'imagerie à partir de l'ensemble d'images histopathologiques colorées, les caractéristiques d'imagerie comprenant des caractéristiques de texture et/ou d'intensité indiquant le paysage structurel et immunitaire de la tumeur ;

l'obtention de données contextuelles correspondant aux un ou plusieurs échantillons de tissu ;

dans une infrastructure de réseau neuronal, le traitement des données d'expression génique obtenues par l'intermédiaire d'une ou plusieurs couches de réseau neuronal d'expression génique configurées pour effectuer une analyse de données moléculaires et traiter simultanément, (i) les caractéristiques d'imagerie déterminées par l'intermédiaire d'une ou plusieurs couches de réseau neuronal de caractéristiques d'imagerie configurées pour extraire des informations visuelles structurelles et immunitaires, et (ii) les données contextuelles par l'intermédiaire d'une ou plusieurs couches de réseau neuronal de caractéristiques contextuelles configurées pour incorporer des aspects contextuels des échantillons de tissu ;

l'intégration, dans l'infrastructure de réseau neuronal, des sorties de la ou des couches de réseau neuronal d'expression génique, de la ou des couches de réseau neuronal de caractéristiques d'imagerie, et de la ou des couches de réseau neuronal de caractéristiques contextuelles pour produire une sortie de réseau neuronal intégrée qui encapsule les caractéristiques moléculaires, visuelles et contextuelles de la tumeur ; et

l'application d'une fonction de prédiction à la sortie de réseau neuronal intégrée pour dériver un score d'infiltration immunitaire pour les un ou plusieurs échantillons de tissu, dans lequel le score indique la composition de cellules immunitaires dans la tumeur.

**FIG. 1**

EP 3 833 777 B1

FIG. 2

**A**

RNA extraction & sequencing → RNA expression gene set

Haemotoxylin and Eosin (H&E) staining → Visual texture feature extraction

NEXT

Immunohistochemistry (IHC) for immune lineage markers → Expert pathology review

Predict

Primary tumor tissue block FFPE

61 samples

**FIG. 3A**

**B**

CD4 T cells

CD8 T cells

CD20 B cells

CD56 NK cells

CD68 Macrophages

H&E image

**FIG. 3B**

Predicting relative immune proportion

**FIG. 4**

FIG. 5A

FIG. 5B

FIG. 6

FIG. 7

800

RECEIVE PRE-PROCESSED GENE EXPRESSION DATA
(e.g., RNA SEQUENCE DATA ON SELECTED GENE(S)
FILTERED USING A GENE LIST AND FILTERED
BASED ON TPM VALUES)

EXTRACT INITIAL GENE EXPRESSION FEATURES
(e.g., RNA-seq FEATURES)

RNA MODULE APPLIES NEURAL NETWORK FRAMEWORK
TO GENE EXPRESSION DATA (e.g., RNA-seq FEATURES)

PREDICTION MODULE GENERATES PREDICTED
PERCENTAGE PROPORTIONS OF IMMUNE
CELLS OR TOTAL FRACTION OF IMMUNE INFILTRATION

FIG. 8

900

```
┌─────────────────────────┐   ┌─────────────────────────┐
│  RECEIVE PRE-PROCESSED   │   │  RECEIVE PRE-PROCESSED   │
│  GENE EXPRESSION DATA    │   │   IMAGE DATA (e.g., H&E  │
│ (e.g., RNA SEQUENCE DATA │   │      STAINED IMAGES)     │
│   ON SELECTED GENE(S))   │   │                          │
└─────────────────────────┘   └─────────────────────────┘
             │                             │
             ▼                             ▼
┌─────────────────────────┐   ┌─────────────────────────┐
│      RNA MODULE          │   │    IMAGING MODULE        │
│  TRANSFORMS RNA DATA     │   │  TRANSFORMS IMAGING      │
│   IN NEURAL NETWORK      │   │   FEATURES IN NEURAL     │
│       LAYER(S)           │   │    NETWORK LAYER(S)      │
└─────────────────────────┘   └─────────────────────────┘
             │                             │
             ▼                             ▼
┌─────────────────────────────────────────────────────────┐
│  INTEGRATION MODULE PERFORMS INTEGRATION ON              │
│       RECEIVED GENE EXPRESSION DATA                      │
│          AND IMAGING FEATURES DATA                       │
└─────────────────────────────────────────────────────────┘
                            │
                            ▼
┌─────────────────────────────────────────────────────────┐
│  PREDICTION MODULE PREDICTION MODULE GENERATES           │
│   PREDICTED PERCENTAGE PROPORTIONS OF IMMUNE             │
│  CELLS OR TOTAL FRACTION OF IMMUNE INFILTRATION          │
│       (e.g., APPLYING SOFTMAX FUNCTION)                  │
└─────────────────────────────────────────────────────────┘
```

# FIG. 9

1000

RECEIVE PRE-PROCESSED
GENE EXPRESSION DATA
(e.g., RNA SEQUENCE DATA
ON SELECTED GENE(S))

RECEIVE PRE-PROCESSED
IMAGE DATA (e.g., H&E
STAINED IMAGES)

RNA MODULE TRANSFORMS
RNA DATA IN NEURAL
NETWORK LAYER(S)

IMAGING MODULE
TRANSFORMS IMAGING
FEATURES IN NEURAL
NETWORK LAYER(S)

CONTEXTUAL
CLASSIFICATION
DATA

INTEGRATION MODULE PERFORMS INTEGRATION ON
RECEIVED GENE EXPRESSION DATA, IMAGING FEATURES
DATA, AND CONTEXTUAL DATA

PREDICTION MODULE PREDICTION MODULE GENERATES
PREDICTED PERCENTAGE PROPORTIONS OF IMMUNE CELLS
OR TOTAL FRACTION OF IMMUNE INFILTRATION
(e.g., APPLYING SOFTMAX FUNCTION)

FIG. 10

**FIG. 11**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62715079

**Non-patent literature cited in the description**

- **SAFOORA YOUSEFI et al.** Predicting clinical outcomes from large scale cancer genomic profiles with deep survival models. *SCIENTIFIC REPORTS*, 15 September 2017, vol. 7 (1)